# EUROPEAN PATENT APPLICATION

(11) **EP 4 052 706 A1**
(43) Date of publication of application: **07.09.2022**
(21) Application number: 20883564.5
(22) Date of filing: 29.10.2020
(51) Int. Cl.: A61K 31/4439, A61K 31/444, A61K 31/4709, A61P 25/00, C07D 413/12, C07D 498/04, C07D 413/14, C07D 401/12

(54) **COMPOSITION FOR PREVENTING OR INHIBITING AXONAL DEGENERATION**

(30) Priority: 31.10.2019 KR 20190137447
(71) Applicant: Checkmate Therapeutics Inc., Seoul 03185 (KR)
(72) Inventor: KIM, Hyun Seok, Goyang-si Gyeonggi-do 10360 (KR); JUNG, Hosung, Seoul 08536 (KR); LEE, Jooyoung, Seoul 07552 (KR)
(74) Representative: Müller, Christian Stefan Gerd
(86) International application number: PCT/KR2020/014946
(87) International publication number: WO 2021/086076

(57) **Abstract**

The present invention relates to compositions capable of preventing or inhibiting axonal degeneration and effectively preventing, almeliorating, or treating various neurological diseases caused by the axonal degeneration.

## Description

### Technical Field

The present invention relates to a composition capable of preventing or inhibiting axonal degeneration and effectively preventing, ameliorating or treating various neurological diseases caused by axonal degeneration.

### Background Art

It is known that axonal degeneration is a direct cause of traumatic brain injury and peripheral neuropathy, and also precedes neuronal death in Alzheimer's disease, Parkinson's disease, Huntington's disease, multiple sclerosis, amyotrophic lateral sclerosis (ALS), and glaucoma (Eric Verdin, Science 2016 PMID 26785480; Conforti et al., Nature Rev Neurosci, 2014 PMID 24840802). Thus, the inhibition of axonal degeneration can be an effective method for preventing or treating these various brain-nervous system diseases.

In order to study axonal degeneration, the Wallerian degeneration model has been most widely used, in which mouse neurons are directly axotomized and which can track a process in which the injured axons are degenerated for 36 to 44 hours (Conforti et al., Nature Rev Neurosci, 2014 PMID 24840802). Genetic mutations that delay the death of injured axons by more than 10 days in the Wallerian degeneration model have been reported. First, the slow Wallerian degeneration protein (WLD^{S}) creates a chimeric fusion protein variant containing nicotinamide mononucleotide adenyltransferase (NMNAT1) originally present in the nucleus, resulting in the expression of NMNAT1 in the axon, and plays a role in synthesizing NAD+ from nicotinamide mononucleotide (NMN) and ATP in the axon. Second, there is SARM1 (Sterile Alpha and TIR Motif Containing 1) knockout. It is known that SARM1 acts to degrade NAD+ into nicotinamide and ADP-ribose (ADPR) through the TIR domain (Essuman et al., Neuron 2017 PMID 28334607), and plays a role in promoting axonal degeneration by depleting axonal NAD+. SARM1 has been found to promote axonal degeneration through MKK4-JNK as a downstream signaling in a traumatic axonal injury model (Yang et al., Cell 2015, PMID 25594179). In addition, it is known that SARM1 promotes JNK phosphorylation, and as a result, promotes immune responses around injured neurons (Wang et al., Cell Reports 2018, PMID 29669278). In addition to WLD^{S} and SARM1, PHR1 knockout is also known to contribute to the preservation of NAD+ by regulating NMNAT2 (Gerdts et al., Neuron 2016 PMID 26844829). These genetic evidences suggest that generation and preservation of NAD+ in axons have an inhibitory effect on axonal degeneration.

As another mechanism of axonal degeneration, the NMN hypothesis has been proposed. The NMN hypothesis is based on several important experimental evidences. First, it is known that FK866, which inhibits the enzyme that converts nicotinamide to NMN, has an effect of partially inhibiting axonal degeneration (Di Stefano et al., Cell Death Diff 2015, PMID 25323584). In addition, it has been found that artificial expression of bacterial NMN deamidase, an enzyme that converts NMN to nicotinic acid mononucleotide (NaMN), exhibits an axon protective effect similar to that of WLD^{S}. These two experimental evidences suggest that NMN accumulation promotes axonal degeneration, and a method of inhibiting NMN accumulation has the effect of inhibiting axonal degeneration (Gerdts et al., Neuron 2016 PMID 26844829).

The two models do not necessarily conflict with each other, and are consistent with each other in that NAD+ is degraded by SARM1 and the like into nicotinamide which is converted into NMN, and in that NMN has the ability to directly activate SARM1 (Zhao et al., iScience 2019 PMID 31128467). Taking the above two models together, it is predicted that axonal degeneration may be inhibited by preserving NAD+ or inhibiting NMN accumulation by methods such as SARM1 inhibition, and when the above two mechanisms are satisfied, the inhibitory effect on axonal degeneration will be increased.

### DISCLOSURE

### Technical Problem

An object of the present invention is to provide a pharmaceutical composition for preventing or inhibiting axonal degeneration.

Another object of the present invention is to provide a pharmaceutical composition for preventing or treating neurological diseases caused by axonal degeneration.

However, objects to be achieved by the present invention are not limited to the above-mentioned objects, and other objects not mentioned herein will be clearly understood by those of ordinary skill in the art from the following description.

### Technical Solution

As used herein, the term "halogen" refers to fluorine, chlorine, bromine or iodine, unless otherwise specified.

As used herein, the term "C₁-C₆ alkyl" refers to a linear or branched hydrocarbon radical having 1 to 6 carbon atoms, unless otherwise specified. Examples thereof include, but are not limited to, methyl, ethyl, propyl, isopropyl, n-butyl, sec-butyl, t-butyl, n-pentyl, n-hexyl, and the like.

As used herein, the term "C₁-C₆ alkoxy" refers to a linear or branched hydrocarbon radical having 1 to 6 carbon atoms and connected to oxygen, unless otherwise specified. Examples thereof include, but are not limited to, methoxy, ethoxy, propoxy, isobutoxy, n-butoxy, sec-butoxy, t-butoxy, pentoxy and hexoxy.

As used herein, the term "C₃-C₇ cycloalkoxy" refers to a cyclic hydrocarbon radical having 3 to 6 carbon atoms and connected to oxygen, unless otherwise specified. Examples thereof include, but are not limited to, cyclopropoxy, cyclobutoxy, cyclopentoxy, cyclohexoxy and cycloheptoxy.

As used herein, the term "C₃-C₇ cycloalkyl" refers to a cyclic hydrocarbon radical having 3 to 7 carbon atoms, unless otherwise specified. Examples thereof include, but are not limited to, cyclopropyl, cyclobutyl, cyclopentyl, cyclohexyl and cycloheptyl.

As used herein, the term "heterocycloalkyl having 5 to 7 nuclear atoms" refers to a 5- to 7-membered cyclic radical containing 1 to 3 optionally selected from among N, O, S, SO and SO₂, unless otherwise specified. Examples thereof include, but are not limited to, tetrahydrofuran-3-yl, tetrahydro-2H-pyran-4-yl, tetrahydro-2H-pyran-3-yl, oxepan-4-yl, oxepan-3-yl, piperidin-1-yl, piperidin-3-yl, piperidin-4-yl, piperazin-1-yl, morpholin-4-yl, thiomorpholin-4-yl, 1,1-dioxide thiomorpholin-4-yl, pyrrolidin-1-yl, pyrrolidin-3-yl, azepan-1-yl, azepan-3-yl and azepan-4-yl.

As used herein, the term "C₆-C₁₄ aryl" refers to a mono- or poly-cyclic carbocyclic ring system containing 6 to 14 carbon atoms and having one or more fused or non-fused aromatic rings, unless otherwise specified, and examples of aryl include, but are not limited to, phenyl, naphthyl, tetrahydronaphthyl, indenyl and andracenyl.

As used herein, the term "heteroaryl having 5 to 14 nuclear atoms" refers to a 5- to 14-membered monocyclic or bicyclic or higher aromatic group containing one or more (e.g., 1 to 4) heteroatoms selected from among O, N and S, unless otherwise specified. Examples of monocyclic heteroaryl include, but are not limited to, thiazolyl, oxazolyl, thiophenyl, furanyl, pyrrolyl, imidazolyl, benzo[d]oxazolyl, isoxazolyl, oxazolopyridinyl, pyrazolyl, triazolyl, thiazolyl, benzo[d]thiazolyl, thiadiazolyl, tetrazolyl, oxadiazolyl, pyridinyl, pyridazinyl, pyrimidinyl, pyrazinyl, quinolinyl, naphthoxazolyl, and the like. Examples of bicyclic heteroaryl include, but are not limited to, indolyl, benzothiophenyl, benzofuranyl, benzimidazolyl, benzoxazolyl, benzisoxazolyl, benzthiazolyl, benzthiadiazolyl, benztriazolyl, quinolinyl, isoquinolinyl, purinyl, furopyridinyl, carbazolyl, dibenzofuranyl, dibenzothiophenyl, and the like.

As used herein, the term "C₅-C₁₄ non-aromatic fused polycyclic ring" refers to a group in which at least two rings are fused with each other and which contains only 6 to 14 carbon atoms as ring forming atoms and has non-aromacity in the entire molecule. Examples thereof include, but are not limited to, fluorenyl, etc.

As used herein, the term "non-aromatic fused heteropolycyclic ring having 5 to 14 nuclear atoms" refers to a group in which at least two rings are fused with each other and which contain, as ring forming atoms, one or more (e.g., 1 to 4) heteroatoms selected from among N, O and S, in addition to carbon, and have non-aromacity in the entire molecule which is 5- to 14-membered. Examples of the non-aromatic fused heteropolycyclic ring include, but are not limited to, indolinyl, isoindolinyl, tetrahydroquinolinyl, tetrahydroisoquinolinyl, 2,3-dihydro-1H-benzo[d]imidazolyl, 2,3-dihydrobenzo[d]oxazolyl, 2,3-dihydrobenzo[d]thiazolyl, 3,3a-dihydropyrazolo[1,5-a]pyrimidinyl, 2,3,3a,7a-tetrahydrobenzo[d]thiazolyl and 2,3,3a,7a-tetrahydro-1H-isoindolyl.

As used herein, the term "fused ring" refers to a fused aliphatic ring, a fused aromatic ring, a fused heteroaliphatic ring, a fused heteroaromatic ring, or combinations thereof.

One embodiment of the present invention is directed to a pharmaceutical composition for preventing or inhibiting axonal degeneration containing, as an active ingredient, a compound selected from among a compound represented by the following Formula (1), and a pharmaceutically acceptable salt, optical isomer, hydrate and solvate thereof: 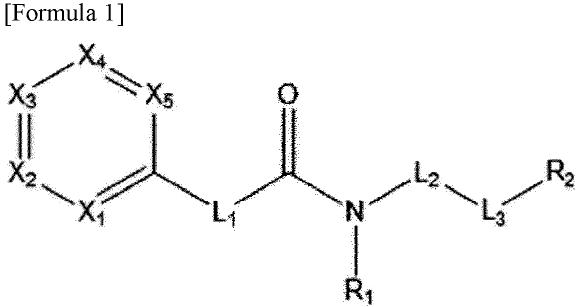 wherein
X₁ to X₅ are each independently N or C(R₃), provided that at least one of X₁ to X₅ is N;
L₁ is a direct bond or a C₁-C₆ alkylene group;
L₂ and L₃ are each independently selected from the group consisting of a direct bond, a C₁-C₆ alkylene group, a C₃-C₇ cycloalkylene group, a heterocycloalkylene group having 5 to 7 nuclear atoms, a C₆-C₁₄ arylene group, a heteroarylene group having 5 to 14 nuclear atoms, a divalent C₅-C₁₄ non-aromatic fused polycyclic group, and a divalent non-aromatic fused heteropolycyclic group having 5 to 14 nuclear atoms;
R₁ is selected from the group consisting of hydrogen, a C₁-C₆ alkyl group, a C₃-C₇ cycloalkyl group, and a heterocycloalkyl group having 5 to 7 nuclear atoms;
R₂ is a group represented by the following Formula 2;
R₃ may be selected from the group consisting of hydrogen, a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₃ is present in a plural number, the plurality of R₃ may be the same as or different from each other, or the plurality of R₃ present adjacent to each other may bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkylene group in L₁ to L₃, the cycloalkylene group, heterocycloalkylene group, arylene group, heteroarylene group, divalent non-aromatic fused polycyclic group and divalent non-aromatic fused heteropolycyclic group in L₂ and L₃, and the alkyl group, cycloalkyl group and heterocycloalkyl group in R₁ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other;
the alkyl group, alkoxy group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₃, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₃ adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other; 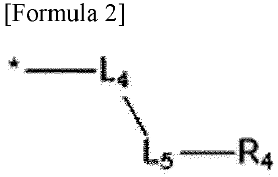 wherein
   ^{∗} denotes a bonding position;
   L₄ and L₅ are each independently selected from the group consisting of a direct bond, a C₁-C₆ alkylene group, a C₆-C₁₄ arylene group, a heteroarylene group having 5 to 14 nuclear atoms, ^{∗}-(CH₂)a-O-(CH₂)b-^{∗}, a carbonyl group (^{∗}-C(=O)-^{∗}), ^{∗}-N(R₅)-^{∗}, and an amide group (^{∗}-C(=O)-N(H)-^{∗} or ^{∗}-N(H)-C(=O)-^{∗});
   a and b are each independently an integer ranging from 0 to 6;
   R₄ is selected from the group consisting of a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, and ^{∗}-N(R₆)(R₇);
   R₅ is selected from the group consisting of hydrogen, a C₁-C₆ alkyl group, a C₃-C₇ cycloalkyl group, and a heterocycloalkyl group having 5 to 7 nuclear atoms;
   R₆ and R₇ are each independently selected from the group consisting of hydrogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, or they bond to each other to form a heterocycloalkyl ring having 5 to 7 nuclear atoms, a heteroaryl ring having 5 to 14 nuclear atoms, or a non-aromatic fused heteropolycyclic ring having 5 to 14 nuclear atoms;
   the alkyl group, cycloalkyl group and heterocycloalkyl group in R₅ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
   the alkylene group, arylene group and heteroarylene group in L₄ and L₅, the cycloalkyl group, heterocycloalkyl group, aryl group, heteroaryl group, non-aromatic fused polycyclic group and non-aromatic fused heteropolycyclic group in R₄, R₆ and R₇, the alkyl group in R₆ and R₇, and the heterocycloalkyl ring, heteroaryl ring and non-aromatic fused heteropolycyclic ring, which are formed by bonding between R₆ and R₇, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other, or the plurality of substituents present adjacent to each other may bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms.

In the present invention, any one of X₁ to X₅ may be N, and the other may be C(R₃).

In the present invention, the compound represented by Formula 1 may be a compound represented by the following Formula 3: 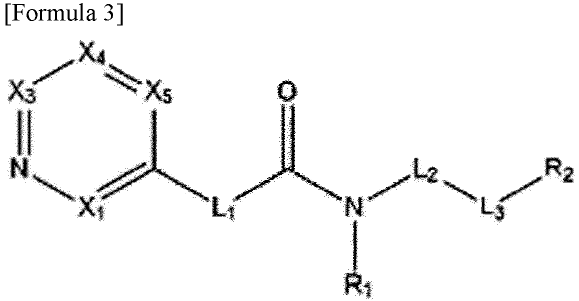 wherein
X₁, X₃ to X₅, L₁ to L₃, R₁ and R₂ are as defined in Formula 1 above.

In the present invention, the compound represented by Formula 1 may be a compound represented by the following Formula 4 or 5: 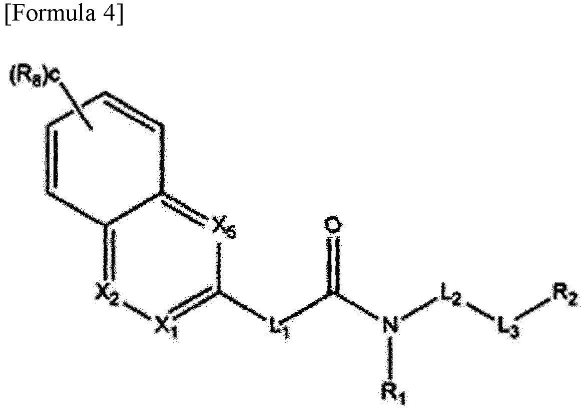 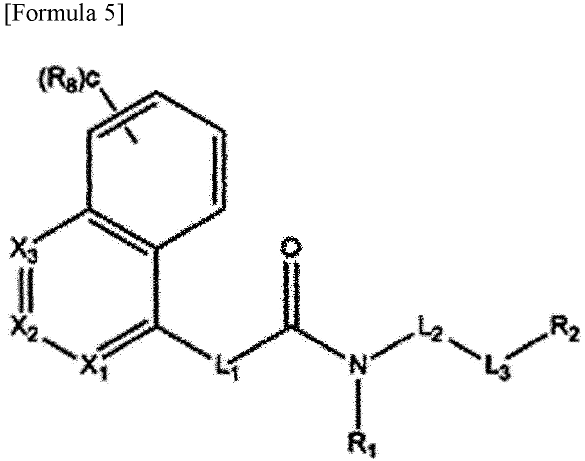 wherein
c is an integer ranging from 0 to 4;
R₈ is selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅ to C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₈ is present in a plural number, the plurality of R₈ are the same as or different from each other;
the alkyl group, alkoxy group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₈ are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other; and
X₁ to X₃, X₅, L₁ to L₃, R₁ and R₂ are as defined in Formula 1 above.

In the present invention, L₁ may be a direct bond or a C₁-C₆ alkylene group unsubstituted or substituted with a C₁-C₆ alkyl group.

In the present invention, the compound represented by Formula 1 may be a compound represented by the following Formula 6: 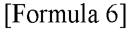 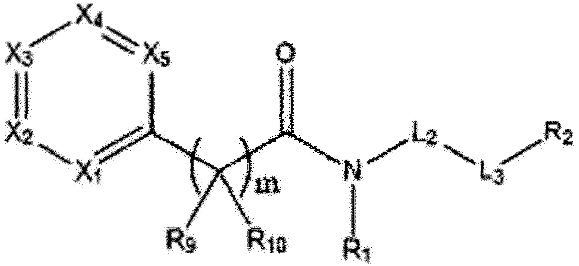 wherein
m is an integer ranging from 0 to 6;
R₉ and R₁₀ are each independently selected from the group consisting of hydrogen, a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, or are each independently preferably hydrogen or a C₁-C₆ alkyl group, and when m is an integer ranging from 2 to 6, the plurality of R₉ are the same as or different from each other and the plurality of R₁₀ are the same as or different from each other;
the alkyl group, cycloalkyl group, heterocycloalkyl group, aryl group, heteroaryl group, non-aromatic fused polycyclic group and non-aromatic fused heteropolycyclic group in R₉ and R₁₀ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, preferably at least one C₁-C₆ alkyl group, and when they are substituted with a plurality of substituents, these substituents are the same as or different from each other; and
X₁ to X₅, L₂, L₃, R₁ and R₂ are as defined in Formula 1 above.

In the present invention, R₁ may be hydrogen or a C₁-C₆ alkyl group unsubstituted or substituted with a C₁-C₆ alkyl group.

In the present invention, L₂ and L₃ may be each independently selected from the group consisting of a direct bond, a C₁-C₆ alkylene group, a C₃-C₇ cycloalkylene group, a heterocycloalkylene group having 5 to 7 nuclear atoms, a C₆-C₁₄ arylene group, and a heteroarylene group having 5 to 14 nuclear atoms, wherein the alkylene group, cycloalkylene group, heterocycloalkylene group, arylene group and heteroarylene group may be each independently substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms.

In the present invention, L₂ may be a direct bond or a C₁-C₆ alkylene group unsubstituted or substituted with a C₁-C₆ alkyl group.

In the present invention, L₃ may be a direct bond or may be selected from the group consisting of a C₃-C₇ cycloalkylene group, a heterocycloalkylene group having 5 to 7 nuclear atoms, a C₆-C₁₄ arylene group, and a heteroarylene group having 5 to 14 nuclear atoms, and the cycloalkylene group, heterocycloalkylene group, arylene group and heteroarylene group in L₃ may be each independently substituted with at least one substituent selected from the group consisting of a halogen, a C1-C6 alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms.

In the present invention, L₃ may be a direct bond, a C₃-C₇ cycloalkylene group or a C₆-C₁₄ arylene group, and the cycloalkylene group and arylene group in L₃ may be each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, and a heteroaryl group having 5 to 14 nuclear atoms, and when they are substituted with a plurality of substituents, these substituents may be the same as or different from each other.

In the present invention, the compound represented by Formula 1 may be a compound represented by the following Formula 7: 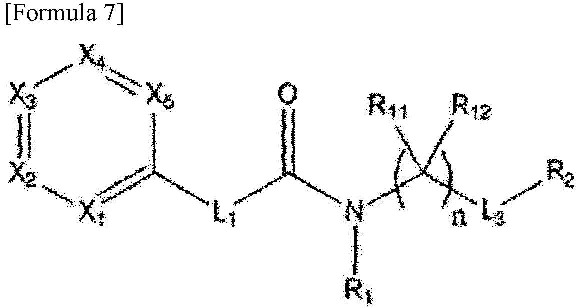 wherein
n is an integer ranging from 0 to 6;
R₁₁ and R₁₂ are each independently selected from the group consisting of hydrogen, a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, preferably hydrogen or a C₁-C₆ alkyl group, and when n is an integer ranging from 2 to 6, the plurality of R₁₁ are the same as or different from each other and the plurality of R₁₂ are the same as or different from each other;
the alkyl group, cycloalkyl group, heterocycloalkyl group, aryl group, heteroaryl group, non-aromatic fused polycyclic group and non-aromatic fused heteropolycyclic group in R₁₁ and R₁₂ are each independently unsubstituted or substituted with at least one substituent from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, preferably at least one C₁-C₆ alkyl group, and when they are substituted with a plurality of substituents, these substituents are the same as or different from each other; and
X₁ to X₅, L₁, L₃, R₁ and R₂ are as defined in Formula 1 above.

In the present invention, the compound represented by Formula 1 may be a compound represented by the following Formula 8 or 9: 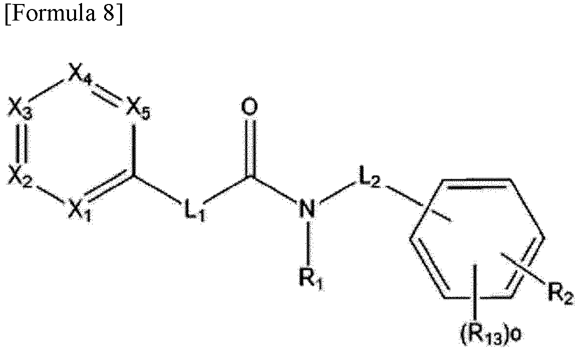 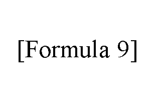 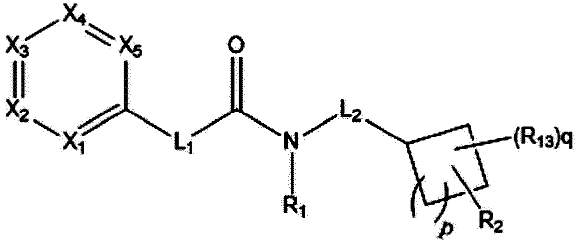 wherein
o and p are each independently an integer ranging from 0 to 4;
q is an integer ranging from 0 to 5;
R₁₃ is selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when R₁₃ is present in a plural number, the plurality of R₁₃ are the same as or different from each other;
the alkyl group, cycloalkyl group, heterocycloalkyl group, aryl group, heteroaryl group, non-aromatic fused polycyclic group and non-aromatic fused heteropolycyclic group in R₁₃ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
X₁ to X₅, L₁, L₂, R₁ and R₂ are as defined in Formula 1 above.

In the present invention, the compound represented by Formula 1 may be a compound represented by the following Formula 10 or 11: 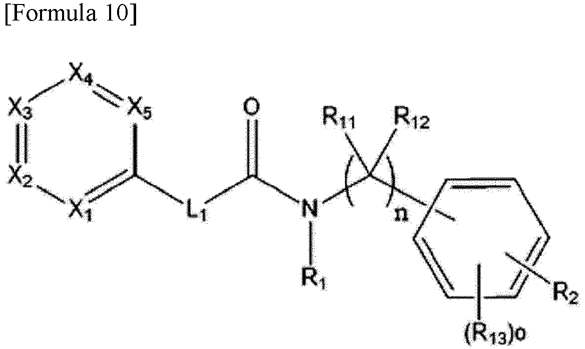 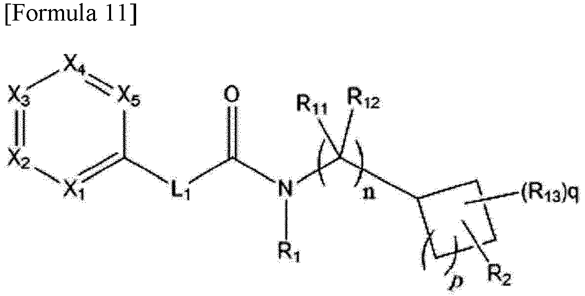 wherein
n is an integer ranging from 0 to 6;
o and p are each independently an integer ranging from 0 to 4;
q is an integer ranging from 0 to 5;
R₁₁ and R₁₂ are each independently selected from the group consisting of hydrogen, a halogen, a C₁-C₆ alkyl group, a C₃-C₇ cycloalkyl group, a heterocycloalkyl group of 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, preferably hydrogen or a C₁-C₆ alkyl group, and when n is an integer ranging from 2 to 6, the plurality of R₁₁ are the same as or different from each other and the plurality of R₁₂ are the same as or different from each other;
R₁₃ is selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when R₁₃ is present in a plural number, the plurality of R₁₃ are the same as or different from each other;
the alkyl group, cycloalkyl group, heterocycloalkyl group, aryl group, heteroaryl group, non-aromatic fused polycyclic group and non-aromatic fused heteropolycyclic group in R₁₁ to R₁₃ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, preferably at least one a C₁-C₆ alkyl group, and when they are substituted with a plurality of substituents, these substituents are the same as or different from each other; and
X₁ to X₅, L₁, R₁ and R₂ are as defined in Formula 1 above.

In the present invention, L₄ and L₅ are each independently selected from the group consisting of a direct bond, a C₁-C₆ alkylene group, a heteroarylene group having 5 to 14 nuclear atoms, ^{∗}-(CH₂)a-O-(CH₂)b-^{∗}, and an amide group (^{∗}-C(=O)-N(H)-^{∗} or ^{∗}-N(H)-C(=O)-^{∗}), and the alkylene group and heteroarylene group in L₄ and L₅ are each independently unsubstituted or substituted with least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, preferably at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, and a heteroaryl group having 5 to 14 nuclear atoms, and when they are substituted with a plurality of substituents, these substituents may be the same as or different from each other.

In the present invention, L₄ and L₅ may be each independently selected from the group consisting of a direct bond, a C₁-C₆ alkylene group, oxadiazole, ^{∗}-(CH₂)a-O-(CH₂)b-^{∗}, and an amide group (^{∗}-C(=O)-N(H)-^{∗} or ^{∗}-N(H)-C(=O)-^{∗}), without being limited thereto.

In the present invention, R₄ may be a group represented by the following Formula 12: 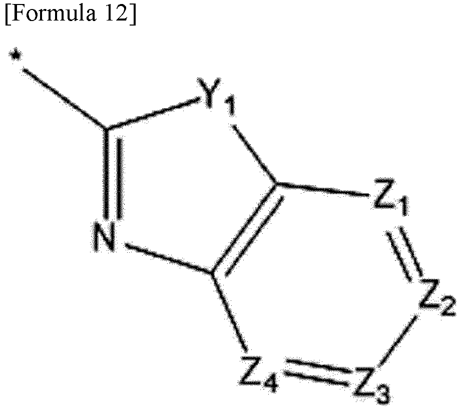 wherein
^{∗} denotes a bonding position;
Y₁ is O or S;
Z₁ to Z₄ are each independently N or C(R₁₄);
R₁₄ may be selected from the group consisting of hydrogen, a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₁₄ is present in a plural number, the plurality of R₁₄ may be the same as or different from each other, or the plurality of R₁₄ present adjacent to each other may bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkyl group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₁₄, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₁₄ present adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other.

In the present invention, R₄ may be represented by any one of the following Formulas a1 to a7, without being limited thereto: 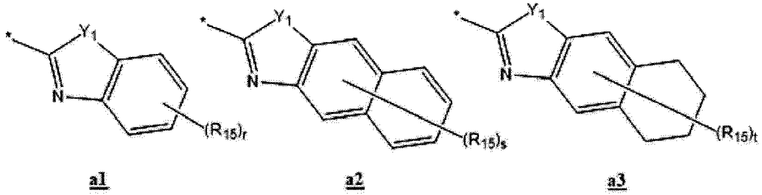 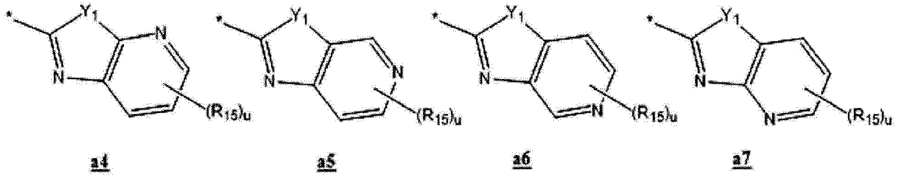 wherein
r is an integer ranging from 0 to 4;
s is an integer ranging from 0 to 6;
t is an integer ranging from 0 to 10;
u is an integer ranging from 0 to 3;
R₁₅ may be selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₁₅ is present in a plural number, the plurality of R₁₅ may be the same as or different from each other, or the plurality of R₁₅ present adjacent to each other may bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkyl group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₁₅, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₁₅ present adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁ to C₆ alkoxycarbonyl group, and a C₁-C₆ alkylsulfonyl group, and when they are substituted with a plurality of substituents, these substituents are the same as or different from each other; and
Y₁ is as defined in Formula 12 above.

In the present invention, R₄ may be selected from the group consisting of a heterocycloalkyl group having 5 to 7 nuclear atoms, a C₆ to C₁₄ aryl group, a heteroaryl group having 5 to 14 nuclear atoms, and ^{∗}-N(R₆)(R₇), and the heterocycloalkyl group, aryl group and heteroaryl group in R₄ may be each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁ to C₆ alkoxycarbonyl group, and a C₁-C₆ alkylsulfonyl group, and when they are substituted with a plurality of substituents, these substituents may be the same as or different from each other.

In the present invention, R₆ and R₇ may bond to each other to form a heterocycloalkyl ring having 5 to 7 nuclear atoms, or a heteroaryl ring having 5 to 14 nuclear atoms, and the heterocycloalkyl ring and heteroaryl ring, which are formed by bonding between R₆ and R₇, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁ to C₆ alkoxycarbonyl group, and a C₁-C₆ alkylsulfonyl group, and when they are substituted with a plurality of substituents, these substituents may be the same as or different from each other.

In the present invention, R₄ may be selected from the group consisting of a phenyl group, a pyridinyl group, a pyrimidinyl group, a thiophenyl group, and a furanyl group, and the phenyl group, pyridinyl group, pyrimidinyl group, thiophenyl group and furanyl group in R₄ are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁ to C₆ alkoxycarbonyl group, and a C₁-C₆ alkylsulfonyl group, and when they are substituted with a plurality of substituents, these substituents may be the same as or different from each other.

In the present invention, R₆ and R₇ may bond to each other to form at least one ring selected from the group consisting of a pyrrolidinyl ring, a pyrazolidinyl ring, an imidazolidinyl ring, a pyrrolyl ring, a pyrazolyl ring, an imidazolyl ring, a triazolyl ring, a piperidinyl ring, a hexahydropyridazinyl ring, a hexahydropyrimidinyl ring, a piperazinyl ring, a triazinanyl ring, a pyridinyl ring, and a pyrimidinyl ring, and the pyrrolidinyl ring, pyrazolidinyl ring, imidazolidinyl ring, pyrrolyl ring, pyrazolyl ring, imidazolyl ring, triazolyl ring, piperidinyl ring, hexahydropyridazinyl ring, hexahydropyrimidinyl ring, piperazinyl ring, triazinanyl ring, pyridinyl ring and pyrimidinyl ring, which are formed by bonding between R₆ and R₇, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁ to C₆ alkoxycarbonyl group, and a C₁-C₆ alkylsulfonyl group, and when they are substituted with a plurality of substituents, these substituents may be the same as or different from each other.

Preferred examples of the compound represented by Formula 1 according to the present invention include, without limitation:
N-(4-(6-methylbenzo[d]oxazol-2-yl)benzyl)nicotinamide;
N-(4-(6-chlorobenzo[d]oxazol-2-yl)benzyl)nicotinamide;
N-(4-(naphtho[2,3-d]oxazol-2-yl)benzyl)nicotinamide;
N-(4-(oxazolo[4,5-c]pyridin-2-yl)benzyl)nicotinamide;
N-(4-(5-(methylsulfonyl)benzo[d]oxazol-2-yl)benzyl)nicotinamide;
N-(4-(5-methylbenzo[d]oxazol-2-yl)benzyl)nicotinamide;
N-(4-(5-ethylbenzo[d]oxazol-2-yl)benzyl)nicotinamide;
N-(4-(6-chlorobenzo[d]oxazol-2-yl)benzyl)-6-(trifluoromethyl)nicotinamide;
N-(4-(6-methylbenzo[d]oxazol-2-yl)benzyl)benzamide;
N-(4-(5,6,7,8-tetrahydronaphtho[2,3-d]oxazol-2-yl)benzyl)nicotinamide;
N-(4-(4-methylbenzo[d]oxazol-2-yl)benzyl)nicotinamide;
N-(4-(5-bromobenzo[d]oxazol-2-yl)benzyl)nicotinamide;
N-(4-(5-(pyridin-3-yl)benzo[d]oxazol-2-yl)benzyl)nicotinamide;
methyl 2-(4-(nicotinamidomethyl)phenyl)benzo[d]oxazole-7-carboxylate;
N-(4-(6-methoxybenzo[d]oxazol-2-yl)benzyl)nicotinamide;
N-(((1r,4r)-4-(6-methylbenzo[d]oxazol-2-yl)cyclohexyl)methyl)nicotinamide;
N-(((1r,4r)-4-(5-(methylsulfonyl)benzo[d]oxazol-2-yl)cyclohexyl)methyl)nicotinamide;
N-(4-(6-methylbenzo[d]oxazol-2-yl)benzyl)-3-(pyridin-3-yl)propanamide;
N-(4-(benzo[d]thiazol-2-yl)benzyl)nicotinamide;
N-(4-(benzo[d]oxazol-2-yl)benzyl)nicotinamide;
N-(4-((4-chloro-1H-pyrazol-1-yl)methyl)benzyl)nicotinamide;
N-(4-(5-methoxybenzo[d]oxazol-2-yl)benzyl)nicotinamide;
N-(4-(6-methylbenzo[d]oxazol-2-yl)phenyl)nicotinamide;
N-(4-(5-(thiophen-2-yl)-1,2,4-oxadiazol-3-yl)phenyl)nicotinamide;
N-(2-(nicotinamido)ethyl)-3-(p-tolyl)-1,2,4-oxadiazole-5-carboxamide;
N-(4-(2-methyl-1H-imidazol-1-yl)benzyl)nicotinamide;
N-(4-(piperidin-1-yl)benzyl)nicotinamide; and
N-(4-(pyridin-2-ylmethoxy)benzyl)nicotinamide.

The present invention also provides a pharmaceutically acceptable salt of the compound represented by Formula 1. The pharmaceutically acceptable salt should have low toxicity to the human body and should not adversely affect the biological activity and physicochemical properties of the parent compound. Examples of the pharmaceutically acceptable salt include, but are not limited to, acid addition salts formed with pharmaceutically acceptable free acids.

Examples of preferred salt forms of the compound according to the present invention include salts with inorganic acids or organic acids. Here, examples of the inorganic acids include hydrochloric acid, sulfuric acid, nitric acid, phosphoric acid, perchloric acid, bromic acid, and the like. In addition, examples of the organic acids include acetic acid, methanesulfonic acid, ethanesulfonic acid, p-toluenesulfonic acid, fumaric acid, maleic acid, malonic acid, phthalic acid, succinic acid, lactic acid, citric acid, citric acid, gluconic acid, tartaric acid, salicylic acid, malic acid, oxalic acid, benzoic acid, embonic acid, aspartic acid, glutamic acid, and the like. Examples of organic bases that may be used in the preparation of organic base addition salts include tris(hydroxymethyl)methylamine, dicyclohexylamine, and the like. Examples of amino acids that may be used in the preparation of amino acid addition salts include natural amino acids such as alanine and glycine. It will be obvious to those skilled in the art that, in addition to the inorganic acids, organic acids, organic bases and amino acids exemplified above, other acids or bases may be used.

The salt may be prepared by a conventional method. For example, the salt may be prepared by dissolving the compound of Formula 1 or 18 in a water-miscible solvent such as methanol, ethanol, acetone, or 1,4-dioxane, and then adding a free acid or a free base thereto, followed by crystallization.

Meanwhile, since the compounds according to the present invention may each have an asymmetric carbon center, they may exist as R or S isomers or racemic mixtures, and these optical isomers and mixtures may all be included in the scope of the present invention.

In addition, a hydrate or solvate of the compound of Formula 1 may be included in the scope of the present invention.

In the present invention, "prevention or inhibition of axonal degeneration" may include, without limitation, (i) inhibiting or preventing axonal degeneration in a subject diagnosed with or likely to develop a neurodegenerative disease, and (ii) inhibiting or preventing axonal degeneration in a subject who has already had a neurodegenerative disease or symptoms thereof. Prevention of axonal degeneration includes, without limitation, reducing or inhibiting axonal degeneration in which the axon is completely or partially reduced.

Another embodiment of the present invention is directed to a pharmaceutical composition for preventing or treating neurological diseases caused by axonal degeneration containing, as an active ingredient, the compound provided in the present invention.

In the present invention, the neurological diseases caused by axonal degeneration may be, without limitation, (i) nervous system diseases, (ii) nervous system injury caused by physical, mechanical or chemical trauma; (iii) pain; (iv) vision-related neurodegeneration; (v) memory loss; or (vi) psychiatric disorders.

In the present invention, non-limiting examples of (i) the nervous system diseases include amyotrophic lateral sclerosis (ALS), trigeminal neuralgia, glossopharyngeal neuralgia, Bell's Palsy, myasthenia gravis, diabetic neuropathy, HIV neuropathy, muscular dystrophy, progressive muscular atrophy, primary lateral sclerosis (PLS), pseudobulbar palsy, progressive bulbar palsy, spinal muscular atrophy, inherited muscular atrophy, intervertebral disc syndrome, cervical spondylosis, nerve root and plexus disorders, thoracic outlet syndrome, peripheral neuropathies, porphyria, Alzheimer's disease, Huntington's disease, Parkinson's disease, Parkinson's-plus diseases, multiple system atrophy, progressive supranuclear palsy, corticobasal degeneration, dementia with Lewy bodies, frontotemporal dementia, demyelinating diseases, Guillain-Barre syndrome, multiple sclerosis, Charcot-Marie-Tooth disease, prion disease, Creutzfeldt-Jakob disease, Gerstmann-Straussler-Scheinker syndrome (GSS), fatal familial insomnia (FFI), bovine spongiform encephalopathy, Pick's disease, epilepsy, and AIDS demential complex.

In the present invention, non-limiting examples of (ii) the nervous system injury caused by physical, mechanical or chemical trauma include injuries caused by exposure to toxic compounds, heavy metals, industrial solvents, drugs, chemotherapy agents, chemotherapy-induced peripheral neuropathy, drug delivery systems, HIV drug therapy, cholesterol lowering drugs, heart or blood pressure therapeutic agents and metronidazole, or injuries caused by burns, wounds, surgery, accident, ischemia, long-term exposure to low temperatures, stroke, intracranial hemorrhage or cerebral hemorrhage.

In the present invention, non-limiting examples of (iii) the pain include chronic pain, fibromyalgia, spinal pain, carpel tunnel syndrome, pain from cancer, arthritis, sciatica, headache, pain from surgery, muscle spasms, back pain, visceral pain, pain from an accident, dental pain, neuralgia, nerve inflammation or injury, shingles, herniated disc, torn ligament, and diabetes.

In the present invention, non-limiting examples of (iv) the vision-related neurodegeneration include glaucoma, lattice dystrophy, retinitis pigmentosa, age-related macular degeneration (AMD), photoreceptor degeneration associated with wet or dry AMD, other retinal degeneration, Leber's hereditary optic neuropathy (LHON), optic nerve drusen, optic neuropathy, and optic neuritis. Here, examples of the glaucoma include, but are not limited to, primary glaucoma, low-tension glaucoma, primary angle-closure glaucoma, acute angle-closure glaucoma, chronic angle-closure glaucoma, intermittent angle-closure glaucoma, chronic open-angle closure glaucoma, pigmentary glaucoma, exfoliation glaucoma, developmental glaucoma, secondary glaucoma, phacogenic glaucoma, glaucoma secondary to intraocular hemorrhage, traumatic glaucoma, neovascular glaucoma, drug-induced glaucoma, toxic glaucoma, and glaucoma caused by intraocular tumors, retinal detachment, chemical burns of the eye, or iris atrophy.

In the present invention, non-limiting examples of (vi) the psychiatric disorders include schizophrenia, delusional disorder, schizoaffective disorder, schizopheniform, shared psychotic disorder, psychosis, paranoid personality disorder, schizoid personality disorder, borderline personality disorder, anti-social personality disorder, narcissistic personality disorder, obsessive-compulsive disorder, delirium, dementia, mood disorders, bipolar disorder, depression, stress disorder, panic disorder, agoraphobia, social phobia, post-traumatic stress disorder, anxiety disorder, and impulse control disorders.

Among the diseases listed above in the present invention, in particular, "chemotherapy-induced peripheral neuropathy (CIPN)" is a serious side effect that occurs frequently after anticancer chemotherapy, and makes it unavoidable to reduce the dose of anticancer drugs or stop drug treatment. CIPN exacerbates symptoms and adversely affects the patient's quality of life. It has been reported that 30 to 40 percent of patients receiving anticancer chemotherapy experience CIPN side effects. CIPN can be caused by various anticancer agents, examples of which include, but are not limited to, Ixabepilone (Ixempra Kit), arsenic trioxide (Trisenox), cytarabine (Cytosar-U, Depocyt, generics), etoposide, hexamethylmelamine (altretamine [Hexalen]), Ifosfamide (Ifex, generics), methotrexate (Trexall, generics), procarbazine (Matulane), vinblastine, and the like. The most common types of anticancer agents that cause CIPN include, but are not limited to, platinum compounds (e.g., cisplatin, carboplatin, and oxaliplatin), vincristine, Taxane-based anticancer agents (e.g., docetaxel and paclitaxel), epothilones (ixabepilone), bortezomib (Velcade), thalidomide (Thalomid), and lenalidomide.

The pharmaceutical composition of the present invention may further contain at least one of nicotinic acid riboside (NAR) represented by the following Formula 13 and nicotinic acid mononucleotide (NAMN) represented by the following Formula 14, thereby preventing toxicity caused by NAD+ reduction or exhibiting a synergistic effect on inhibiting axonal degeneration:

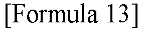

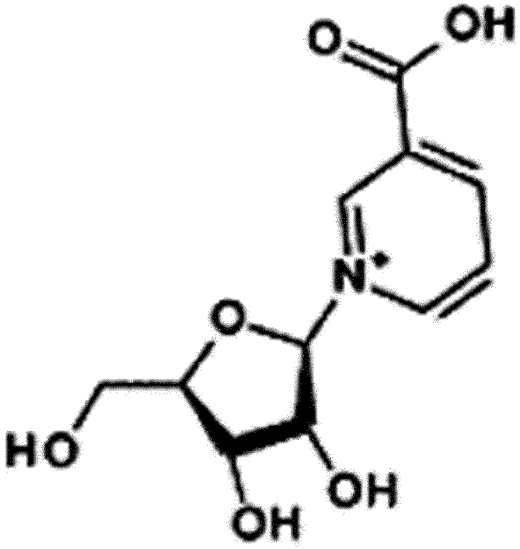

### [Example 14]

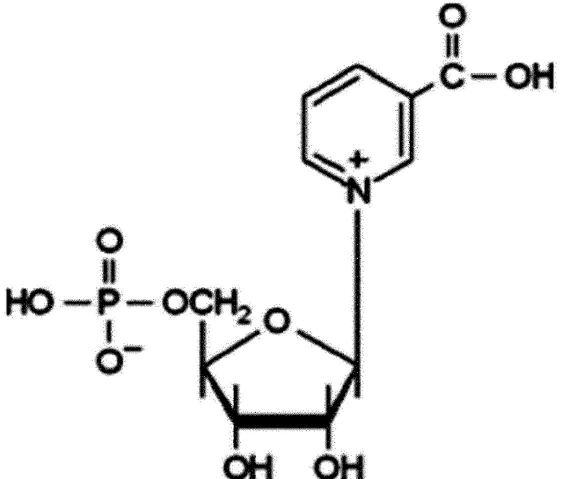

In the present invention, "treatment" and "amelioration" may include, without limitation, any action that alleviates or beneficially changes various neurological diseases caused by axonal degeneration, by inhibiting axonal degeneration using a pharmaceutical composition.

In the present invention, "prevention" may include, without limitation, any action that blocks, suppresses or delays symptoms of various neurological diseases caused by axonal degeneration, by preventing axonal degeneration using the pharmaceutical composition of the present invention.

In the present invention, the pharmaceutical composition may be in the form of capsules, tablets, granules, injections, ointments, powders or beverages, and the pharmaceutical composition may be for administration to humans.

For use, the pharmaceutical composition of the present invention may be formulated in the form of, but not limited to, oral preparations, such as powders, granules, capsules, tablets, and aqueous suspensions, as well as external preparations, suppositories, and sterile injectable solutions, according to the respective conventional methods. The pharmaceutical composition of the present invention may contain pharmaceutically acceptable carriers. Examples of the pharmaceutically acceptable carriers that may be used for oral administration include binders, lubricants, disintegrants, excipients, solubilizers, dispersants, stabilizers, suspending agents, colorants, flavorings, and the like, and examples of the pharmaceutically acceptable carriers that may be used for injection include buffers, preservatives, analgesics, solubilizers, isotonic agents, stabilizers, and the like. Examples of the pharmaceutically acceptable carriers that may be used for topical administration include bases, excipients, lubricants, preservatives, and the like. The formulation of the pharmaceutical composition of the present invention may be prepared in various ways by mixing with the aforementioned pharmaceutically acceptable carriers. For example, for oral administration, the pharmaceutical composition may be prepared in the form of tablets, troches, capsules, elixir, suspensions, syrups, wafers, and for injection, the pharmaceutical composition may be prepared in a unit dose form or prepared to be contained in a multi-dose container. In addition, the pharmaceutical composition may be formulated as solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further contain a filler, an anticoagulant, a lubricant, a wetting agent, a fragrance, an emulsifier, a preservative, or the like.

The routes of administration of the pharmaceutical composition according to the present invention include, but are not limited to, oral, intravenous, intramuscular, intra-arterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, "parenteral" includes subcutaneous, transdermal, intravenous, intramuscular, intra-articular, intra-synovial, intrasternal, intradural, intraocular, intra-lesional and intra-cranial injection or infusion techniques. The pharmaceutical composition of the present invention may also be formulated as suppositories for intrarectal administration.

The pharmaceutical composition of the present invention may vary depending on various factors, including the activity of specific compound used, the patient's age, body weight, general health, sex, and diet, the time of administration, the route of administration, excretion rate, the drug content, and the severity of a specific disease to be prevented or treated. The dosage of the pharmaceutical composition may vary depending on the patient's condition and body weight, the severity of the disease, the form of drug, and the route and duration of administration, but may be suitably selected by a person skilled in the art, and may be administered at a dose of 0.0001 to 1,000 mg/kg/day or 0.001 to 500 mg/kg/day. The pharmaceutical composition may be administered once or several times a day. The dosage is not intended to limit the scope of the present invention in any way. The pharmaceutical composition according to the present invention may be formulated as pills, sugar-coated tablets, capsules, liquids, gels, syrups, slurries, or suspensions.

The pharmaceutical composition of the present invention may be used alone or in combination with methods using surgery, radiotherapy, hormone therapy, chemotherapy, and biological response modifiers.

Still another embodiment of the present invention is directed to a method for preventing or inhibiting axonal degeneration including a step of administering, to a subject (e.g., a human) in need of administration, a pharmaceutically effective amount of a compound selected from the compound represented by Formula 1 according to the present invention, and a pharmaceutically acceptable salt, optical isomer, hydrate and solvent thereof.

The prevention or inhibition method according to the present invention may include additionally administering at least one of the nicotinic acid riboside (NAR) represented by Formula 13 and the nicotinic acid mononucleotide (NAMN) represented by Formula 14, thereby preventing toxicity caused by NAD+ reduction or exhibiting a synergistic effect on inhibiting axonal degeneration.

Yet another embodiment of the present invention is directed to a method for preventing or treating neurological diseases caused by axonal degeneration including a step of administering, to a subject (e.g., a human) in need of administration, a pharmaceutically effective amount of a compound selected from the compound represented by Formula 1 according to the present invention, and a pharmaceutically acceptable salt, optical isomer, hydrate and solvent thereof.

The prevention or inhibition method according to the present invention may include additionally administering at least one of the nicotinic acid riboside (NAR) represented by Formula 13 and the nicotinic acid mononucleotide (NAMN) represented by Formula 14, thereby preventing toxicity caused by NAD+ reduction or exhibiting a synergistic effect on inhibiting axonal degeneration.

In the present invention, "administering" means providing a given compound of the present invention to a subject by any suitable method.

In the present invention, the "subject" in need of administration may include both mammals and non-mammals. Here, examples of the mammals include, but are not limited to, humans, non-human primates such as chimpanzees, and other apes and monkey species; livestock animals such as cattle, horses, sheep, goats and pigs; domestic animals such as rabbits, dogs and cats; and laboratory animals, for example, rodents such as rats or mice, and guinea pigs. In addition, examples of the non-mammals in the present invention include, but are not limited to, birds and fish.

In the present invention, the formulation of the compound that is administered as described above is not particularly limited, and the compound of the present invention may be administered as preparations in solid form, preparations in liquid form, or aerosol preparations for inhalation. In addition, it may be administered as preparations in solid form which are intended to be converted, immediately before use, into preparations, for oral or parenteral administration. Forexample, the pharmaceutical composition of the present invention may be formulated for administration in the form of, but not limited to, oral preparations, such as powders, granules, capsules, tablets, and aqueous suspensions, as well as external preparations, suppositories, and sterile injectable solutions.

In addition, in the present invention, pharmaceutically acceptable carriers may be additionally administered together with the compound of the present invention. Here, examples of pharmaceutically acceptable carriers that may be used for oral administration include binders, lubricants, disintegrants, excipients, solubilizers, dispersants, stabilizers, suspending agents, colorants, flavorings, and the like, and examples of pharmaceutically acceptable carriers that may be used for injection include buffers, preservatives, analgesics, solubilizers, isotonic agents, stabilizers, and the like. Examples of pharmaceutically acceptable carriers that may be used for topical administration include bases, excipients, lubricants, preservatives, and the like. The formulation of the compound of the present invention may be prepared in various ways by mixing with the aforementioned pharmaceutically acceptable carriers. For example, for oral administration, the pharmaceutical composition may be prepared in the form of tablets, troches, capsules, elixir, suspensions, syrups, wafers, and for injection, the pharmaceutical composition may be prepared in a unit dose form or prepared to be contained in a multi-dose container. In addition, the pharmaceutical composition may be formulated as solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the composition of the present invention may further contain a filler, an anticoagulant, a lubricant, a wetting agent, a fragrance, an emulsifier, a preservative, or the like.

The routes of administration of the compound according to the present invention include, but are not limited to, oral, intravenous, intramuscular, intra-arterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, "parenteral" includes subcutaneous, transdermal, intravenous, intramuscular, intra-articular, intra-synovial, intrasternal, intradural, intra-lesional and intra-cranial injection or infusion techniques. The pharmaceutical composition of the present invention may also be formulated as suppositories for intrarectal administration.

In the present invention, a "pharmaceutically effective amount" refers to a sufficient amount of an agent to provide a desired biological result. That result can be reduction and/or alleviation of a sign, symptom, or cause of a disease or disorder, or any other desired alteration of a biological system. For example, an effective amount for therapeutic uses is the amount of the compound disclosed herein required to provide a clinically significant reduction in disease. An appropriate "effective" amount in any individual case may be determined by one of ordinary skill in the art using routine experimentation. Accordingly, the expression "effective amount" generally refers to an amount in which an active substance has a therapeutic effect.

The dosage of the compound of the present invention may vary depending on various factors, including the activity of specific compound used, the patient's age, body weight, general health, sex, and diet, the time of administration, the route of administration, excretion rate, the drug content, and the severity of a specific disease to be prevented or treated. The dosage of the compound may vary depending on the patient's condition and body weight, the severity of the disease, the form of drug, and the route and duration of administration, but may be suitably selected by a person skilled in the art, and may be administered at a dose of 0.0001 to 1,000 mg/kg/day or 0.001 to 500 mg/kg/day. The compound may be administered once or several times a day. The dosage is not intended to limit the scope of the present invention in any way. The pharmaceutical composition according to the present invention may be formulated as pills, sugar-coated tablets, capsules, liquids, gels, syrups, slurries, or suspensions.

### Advantageous Effects

It is known that SARM1 promotes JNK phosphorylation, and, as a result, promotes immune responses around injured neurons. The compounds provided in the present invention may effectively prevent or inhibit axonal degeneration by inhibiting the expression of p-JNK, and furthermore, may prevent, ameliorate or treat various neurological diseases caused by axonal degeneration. In addition, the compounds provided in the present invention may also effectively prevent, ameliorate or treat chemotherapy-induced peripheral neuropathy (CIPN) by inhibiting or delaying axonal degeneration caused by anticancer chemotheraoly.

### Brief Description of Drawings

FIG. 1 shows the results of examining changes in the expression levels of JNK and phosphorylated JNK by Western blot assay after treating the gastric cancer cell line MKN1 with the compound according to the present invention in Experimental Example 1.
FIGS. 2 to 12 show the results of examining changes in the expression level of phosphorylated JNK by Western blot analysis after treating the F11 cell line with the compound according to the present invention and the SARM1 activator AP20187 in Experimental Example 2.
FIG. 13 schematically shows an experimental design of Experimental Example 3 of the present invention.
FIG. 14 shows the results of imaging the green fluorescent protein signal of the optic nerve in the cerebral hemisphere by a confocal microscope after treating a tadpole optic nerve axotomy model with the compound according to the present invention and LSN 3154567 and KPT9274 as controls in Experimental Example 3 of the present invention.
FIG. 15 shows the results of measuring the degree of delay in Wallerian degeneration after treating a tadpole optic nerve axotomy model with the compound according to the present invention and LSN 3154567 and KPT9274 as controls in Experimental Example 3 of the present invention.
FIG. 16 shows the results of measuring the axon survival score after treating a tadpole optic nerve axotomy model with the compound according to the present invention in Experimental Example 4 of the present invention.

### Best Mode

One embodiment of the present invention is directed to a pharmaceutical composition for prevention or inhibition of axonal degeneration or for prevention or treatment of neurological diseases caused by axonal degeneration, the pharmaceutical composition containing, as an active ingredient, a compound selected from among a compound represented by the following Formula (1), and a pharmaceutically acceptable salt, optical isomer, hydrate and solvate thereof: 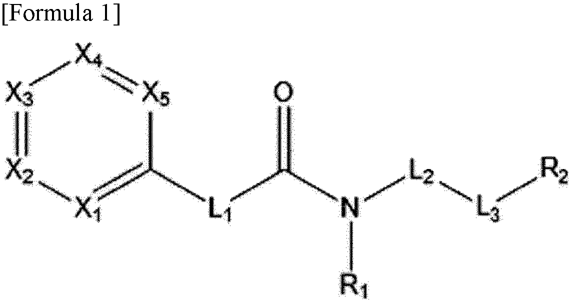 Wherein
X₁ to X₅ are each independently N or C(R₃), provided that at least one of X₁ to X₅ is N;
L₁ is a direct bond or a C₁-C₆ alkylene group;
L₂ and L₃ are each independently selected from the group consisting of a direct bond, a C₁-C₆ alkylene group, a C₃-C₇ cycloalkylene group, a heterocycloalkylene group having 5 to 7 nuclear atoms, a C₆-C₁₄ arylene group, a heteroarylene group having 5 to 14 nuclear atoms, a divalent C₅-C₁₄ non-aromatic fused polycyclic group, and a divalent non-aromatic fused heteropolycyclic group having 5 to 14 nuclear atoms;
R₁ is selected from the group consisting of hydrogen, a C₁-C₆ alkyl group, a C₃-C₇ cycloalkyl group, and a heterocycloalkyl group having 5 to 7 nuclear atoms;
R₂ is a group represented by the following Formula 2;
R₃ may be selected from the group consisting of hydrogen, a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₃ is present in a plural number, the plurality of R₃ may be the same as or different from each other, or the plurality of R₃ present adjacent to each other may bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkylene group in L₁ to L₃, the cycloalkylene group, heterocycloalkylene group, arylene group, heteroarylene group, divalent non-aromatic fused polycyclic group and divalent non-aromatic fused heteropolycyclic group in L₂ and L₃, and the alkyl group, cycloalkyl group and heterocycloalkyl group in R₁ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other;
the alkyl group, alkoxy group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₃, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₃ adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other; 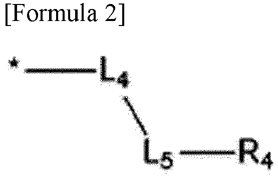 wherein
   ^{∗} denotes a bonding position;
   L₄ and L₅ are each independently selected from the group consisting of a direct bond, a C₁-C₆ alkylene group, a C₆-C₁₄ arylene group, a heteroarylene group having 5 to 14 nuclear atoms, ^{∗}-(CH₂)a-O-(CH₂)b-^{∗}, a carbonyl group (^{∗}**-**C(=O)-^{∗}), ^{∗}-N(R₅)-^{∗}, and an amide group (^{∗}-C(=O)-N(H)-^{∗} or ^{∗}-N(H)-C(=O)-^{∗});
   a and b are each independently an integer ranging from 0 to 6;
   R₄ is selected from the group consisting of a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, and ^{∗}-N(R₆)(R₇);
   R₅ is selected from the group consisting of hydrogen, a C₁-C₆ alkyl group, a C₃-C₇ cycloalkyl group, and a heterocycloalkyl group having 5 to 7 nuclear atoms;
   R₆ and R₇ are each independently selected from the group consisting of hydrogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, or they bond to each other to form a heterocycloalkyl ring having 5 to 7 nuclear atoms, a heteroaryl ring having 5 to 14 nuclear atoms, or a non-aromatic fused heteropolycyclic ring having 5 to 14 nuclear atoms;
   the alkyl group, cycloalkyl group and heterocycloalkyl group in R₅ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
   the alkylene group, arylene group and heteroarylene group in L₄ and L₅, the cycloalkyl group, heterocycloalkyl group, aryl group, heteroaryl group, non-aromatic fused polycyclic group and non-aromatic fused heteropolycyclic group in R₄, R₆ and R₇, the alkyl group in R₆ and R₇, and the heterocycloalkyl ring, heteroaryl ring and non-aromatic fused heteropolycyclic ring, which are formed by bonding between R₆ and R₇, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other, or the plurality of substituents present adjacent to each other may bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms.

### Mode for Invention

Hereinafter, the present invention will be described in more detail with reference to examples. These examples are only for illustrating the present invention in more detail, and it will be obvious to those of ordinary skill in the art that the scope of the present invention according to the subject matter of the present invention is not limited by these examples.

### Examples

### [Preparation Example 11 Preparation of intermediate 1 ((4-(6-methylbenzo[d]oxazol-2-yl)phenyl)methanamine)

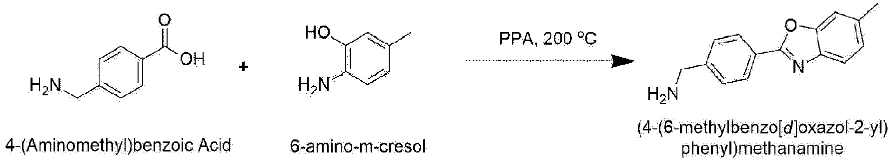

4-(aminomethyl)benzoic acid (1.99 mmol) and 6-amino-m-cresol (1.99 mmol) were dissolved in polyphosphoric acid (PPA, excess) and then refluxed at 200°C for 12 hours. The reaction mixture was dissolved in an excess of distilled water, and the precipitated salt was filtered, dried under vacuum, and used in the next step without further purification.

### [Preparation Example 21 Preparation of intermediate 2 ((4-(6-chlorobenzo[d]oxazol-2-yl)phenyl)methanamine)

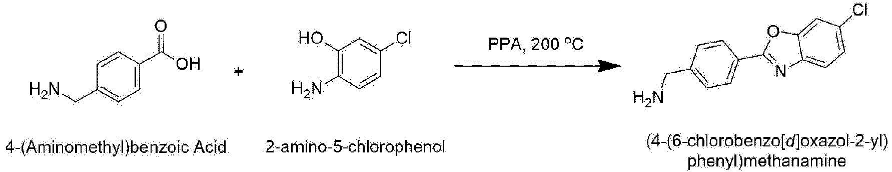

4-(aminoethyl)benzoic acid (1.32 mmol) and 2-amino-5-chlorophenol (1.32 mmol) were dissolved in polyphosphoric acid (PPA, excess) and then refluxed at 200°C for 12 hours. The reaction mixture was dissolved in an excess of distilled water, and the precipitated salt was filtered, dried under vacuum, and used in the next step without further purification.

### [Preparation Example 31 Preparation of intermediate 3 (4-(naphtho[2,3-d]oxazol-2-yl)phenyl)methanamine)

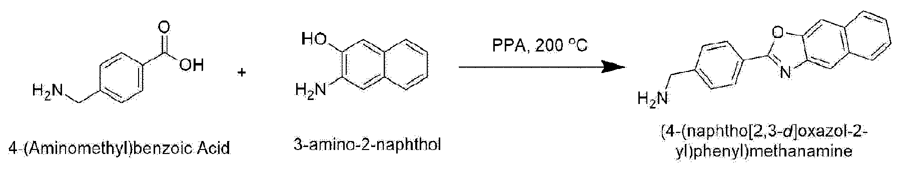

4-(aminoethyl)benzoic acid (1.32 mmol) and 3-amino-2-naphthol (1.32 mmol) were dissolved in polyphosphoric acid (PPA, excess) and then refluxed at 200°C for 12 hours. The reaction mixture was dissolved in an excess of distilled water, and the precipitated salt was filtered, dried under vacuum, and used in the next step without further purification.

### [Preparation Example 41 Preparation of (4-(5-(methylsulfonyl)benzo[d]oxazol-2-yl)phenyl)methanamine

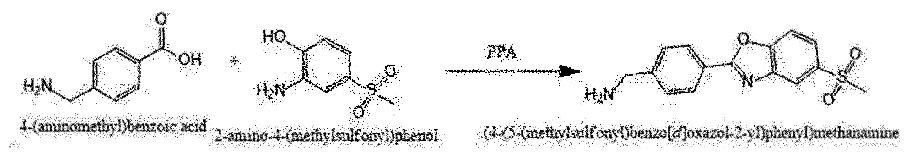

4-(aminomethyl)benzoic acid (1 eq.) and 2-amino-4- (methylsulfonyl)phenol (1 eq.) were placed in a 20-ml vial, and 4 g of polyphosphoric acid (PPA) was added thereto, followed by stirring at 120°C for 12 hours. The reaction mixture was adjusted to pH 7 using a 10% K₂CO₃ aqueous solution and then transferred to a separatory funnel and extracted three times with dichloromethane (MC). The extract was dried over Na₂SO₄ and concentrated under reduced pressure. The residue was used in the next reaction without a separate purification process.

### [Synthesis Example 11 Preparation of N-(4-(6-methylbenzo[d]oxazol-2-yl)benzyl)nicotinamide (A4276)

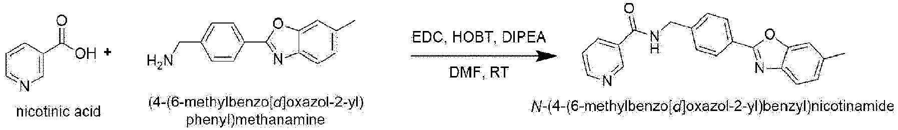

Nicotinic acid (1.06 mmol), the (4-(6-methylbenzo[d]oxazol-2-yl)phenyl)methanamine (1.06 mmol) prepared in Preparation Example 1, (1-(3-dimethylaminopropyl)-3-ethylcarbodiimide hydrochloride (EDC, 2.11 mmol), 1-hydroxybenzotriazole monohydrate (HOBt, 2.11 mmol), and N,N-diisopropylethylamine (DIPEA, 3.17 mmol) were dissolved in dimethylformamide (DMF, 4 ml) and then refluxed overnight at room temperature. Then, the reaction mixture was transferred to a separate funnel, and then an aqueous NH₄Cl solution was added thereto, followed by extraction three times with ethyl acetate (EA). The extract was dried over Na₂SO₄ and concentrated under reduced pressure. The residue was purified using dichloromethane (MC) and hexane (HX) to obtain N-(4-(6-methylbenzo[d]oxazol-2-yl)benzyl)nicotinamide (hereinafter referred to as "A4276") as a white solid compound.

¹H NMR (400 MHz, DMSO-d₆) : δ 9.36 (t, J = 5.6 Hz, 1H), 9.09 (d, J = 1.2 Hz, 1H), 8.74 (dd, J = 4.6, 1.3 Hz, 1H), 8.27 (d, J = 8.0 Hz, 1H), 8.16 (d, J = 8.2 Hz, 2H), 7.67 (d, J = 8.1 Hz, 1H), 7.62 - 7.51 (m, 4H), 7.23 (d, J = 8.0 Hz, 1H), 4.62 (d, J = 5.7 Hz, 2H), 2.47 (s, 3H).

### [Synthesis Example 21 Preparation of N-(4-(6-methylbenzo[d]oxazol-2-yl)benzyl)nicotinamide hydrochloride (A4276H)

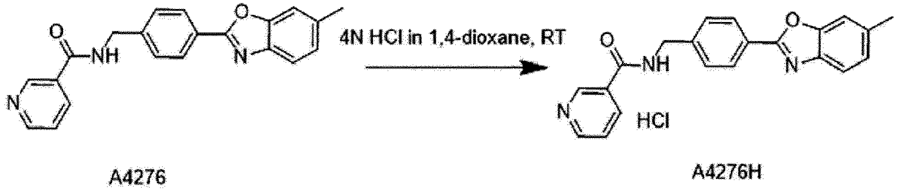

A4276 (1 eq., 4.45 mmol) obtained in Synthesis Example 1 was placed in a 20-ml vial, and 5 ml of a solution 4 M hydrochloric acid (HCl) in 1,4-dioxane was added thereto, followed by stirring at room temperature for 4 hours. The reaction mixture was diluted with ether and stirred at room temperature for 30 minutes. Next, the product was filtered and dried in a vacuum pump to obtain A4276 hydrochloride (A4276H) as a white solid.

¹H NMR (400 MHz, DMSO-d₆) : δ 9.96-9.92 (t,1H), 9.39-9.38 (d, 1H), 9.03-8.90 (m, 2H), 8.17-8.14 (m, 2H), 7.68-7.60 (m, 4H), 7.25 (d, 2H), 4.65-4.63 (d, 2H), 2.47 (s, 3H).

### [Synthesis Example 31 Preparation of N-(4-(6-chlorobenzo[d]oxazol-2-yl)benzyl)nicotinamide (A4266)

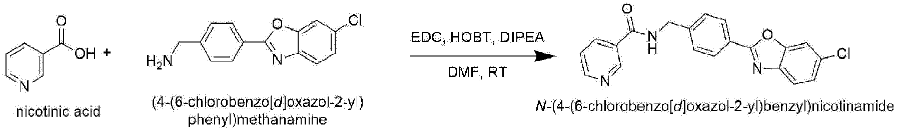

Nicotinic acid (0.77 mmol), the (4-(6-chlorobenzo[d]oxazol-2-yl)phenyl)methanamine (0.77 mmol) prepared in Preparation Example 2, (1-(3-dimethylaminopropyl)-3-ethylcarbodiimide hydrochloride (EDC, 1.54 mmol), 1-hydroxybenzotriazole monohydrate (HOBt, 1.54 mmol), and N,N-diisopropylethylamine (DIPEA, 2.31 mmol) were dissolved in dimethylformamide (DMF, 4 ml) and refluxed overnight at room temperature. Then, the reaction mixture was transferred to a separate funnel, and then an aqueous NH₄Cl solution was added thereto, followed by extraction three times with ethyl acetate (EA). The extract was dried over Na₂SO₄ and concentrated under reduced pressure. The residue was purified using dichloromethane (MC) and hexane (HX) to obtain N-(4-(6-chlorobenzo[d]oxazol-2-yl)benzyl)nicotinamide (hereinafter referred to as "A4266") as a white solid compound.

¹H NMR (400 MHz, DMSO-d₆) : δ 9.37 (t, J = 5.6 Hz, 1H), 9.09 (s, 1H), 8.74 (d, J = 4.0 Hz, 1H), 8.27 (d, J = 7.9 Hz, 1H), 8.17 (d, J = 8.1 Hz, 2H), 8.00 (d, J = 1.3 Hz, 1H), 7.82 (d, J = 8.5 Hz, 1H), 7.62 - 7.51 (m, 3H), 7.47 (d, J = 8.5 Hz, 1H), 4.62 (d, J = 5.6 Hz, 2H).

### [Synthesis Example 41 Preparation of N-(4-(naphtho[2,3-d]oxazol-2-yl)benzyl)nicotinamide (A4265)

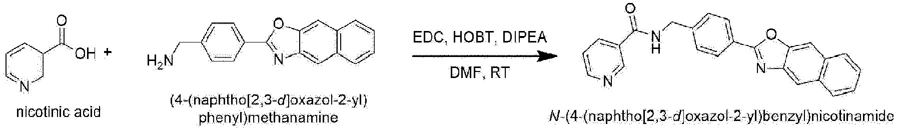

Nicotinic acid (0.73 mmol), the (4-(naphtho[2,3-d]oxazol-2-yl)phenyl)methanamine (0.73 mmol) prepared in Preparation Example 3, 1-(3-dimethylaminopropyl)-3-ethylcarbodiimide hydrochloride (EDC, 1.46 mmol), 1-hydroxybenzotriazole monohydrate (HOBt, 1.46 mmol), and N,N-diisopropylethylamine (DIPEA, 2.19 mmol) were dissolved in dimethylformamide (DMF, 4 ml) and then refluxed overnight at room temperature. Next, the reaction mixture was transferred to a separate funnel, and then an aqueous NH₄Cl solution was added thereto, followed by extraction three times with ethyl acetate (EA). The extract was dried over Na₂SO₄ and concentrated under reduced pressure. The residue was purified using dichloromethane (MC) and hexane (HX) to obtain N-(4-(naphtho[2,3-d]oxazol-2-yl)benzyl)nicotinamide (hereinafter referred to as "A4265") as a white solid compound.

¹H NMR (400 MHz, DMSO-d₆) : δ 9.39 (t, J = 5.5 Hz, 1H), 9.10 (s, 1H), 8.75 (d, J = 3.4 Hz, 1H), 8.34 (s, 1H), 8.31 - 8.24 (m, 4H), 8.09 (t, J = 8.6 Hz, 2H), 7.62 (d, J = 8.1 Hz, 2H), 7.58 - 7.49 (m, 3H), 4.65 (d, J = 5.5 Hz, 2H).

### [Synthesis Example 51 Preparation of N-(4-(oxazolo[4,5-c]pyridin-2-yl)benzyl)nicotinamide (B1471)

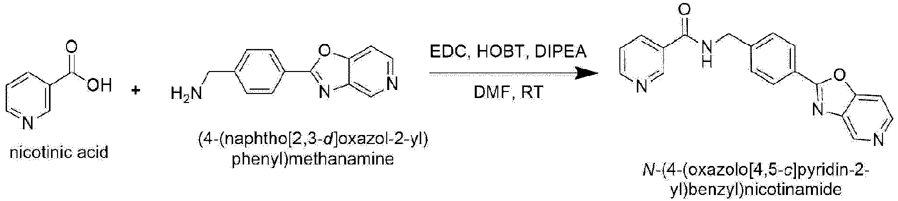

Nicotinic acid (2.44 mmol), 4-(naphtho[2,3-d]oxazol-2-yl)phenyl)methanamine (2.44 mmol), 1-(3-dimethylaminopropyl)-3-ethylcarbodiimide hydrochloride (EDC, 4.87 mmol), 1-hydroxybenzotriazole monohydrate (HOBt, 4.87 mmol), and N,N-diisopropylethylamine (DIPEA, 7.31 mmol) were dissolved in dimethylformamide (DMF, 10 ml) and then refluxed overnight at room temperature. Then, the reaction mixture was transferred to a separate funnel, and then an aqueous NH₄Cl solution was added thereto, followed by extraction three times with ethyl acetate (EA). The extract was dried over Na₂SO₄ and concentrated under reduced pressure. The residue was purified using dichloromethane (MC) and hexane (HX) to obtain N-(4-(oxazolo[4,5-c]pyridin-2-yl)benzyl)nicotinamide (hereinafter referred to as "B1471") as a white solid compound.

¹H NMR (400 MHz, DMSO-d₆): δ 9.39 (t, 1H, J = 5.8 Hz), 9.14 - 9.08 (m, 2H), 8.74 (dd, 1H, J = 4.8, 1.5 Hz), 8.60 (d, 1H, J = 5.5 Hz), 8.29 - 8.25 (m, 1H), 8.22 (d, 2H, J = 8.3 Hz), 7.91 (dd, 1H, J = 5.5, 0.7 Hz), 7.61 (d, 2H, J = 8.3 Hz), 7.55 (dd, 1H, J = 7.9, 4.9 Hz), 4.63 (d, 2H, J = 5.8 Hz).

### [Synthesis Example 61 Preparation of N-(4-(5-(methylsulfonyl)benzo[d]oxazol-2-yl)benzyl)nicotinamide (A4510)

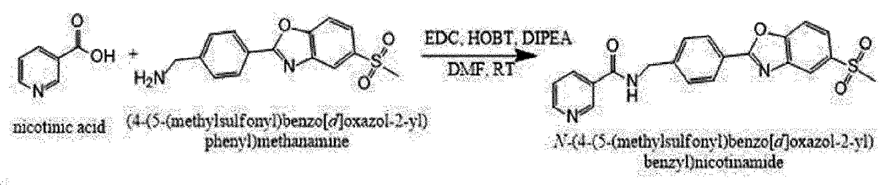

Nicotinic acid (1 eq., 1.41 mmol), the (4-(5- (methylsulfonyl)benzo[d]oxazol-2-yl)phenyl)methanamine (1 eq., 1.41 mmol) prepared in Preparation Example 1, 1-(3-dimethylaminopropyl)-3-ethylcarbodiimide hydrochloride (EDC, 1.1 eq., 1.55 mmol), 1-hydroxybenzotriazole monohydrate (HOBt, 1.1 eq., 1.55 mmol), and N,N-diisopropylethylamine (DIPEA, 3 eq., 4.22 mmol) were dissolved in MC (0.1 M, 14.1 ml) and then refluxed at room temperature overnight. Thereafter, the reaction mixture was transferred to a separatory funnel and distilled water was added thereto, followed by extraction three times with MC. The extract was dried over Na₂SO₄ and concentrated under reduced pressure. The residue was purified by flash column chromatography (5% MeOH in dichloromethane), and then dried in a vacuum pump to obtain N-(4-(5-(methylsulfonyl)benzo[d]oxazole-2-yl)benzyl)nicotinamide (A4510) as a white solid (yield: 66.9%).

¹H NMR (400 MHz, DMSO-d₆) δ 9.35 (t, J = 5.8 Hz, 1H), 9.05 (s, 1H), 8.73 - 8.67 (m, 1H), 8.32 (d, J = 1.5 Hz, 1H), 8.26 - 8.16 (m, 3H), 8.03 (d, J = 8.5 Hz, 1H), 7.96 (dd, J = 8.6, 1.7 Hz, 1H), 7.57 (d, J = 8.1 Hz, 2H), 7.51 (dd, J = 7.9, 4.8 Hz, 1H), 4.59 (d, J = 5.8 Hz, 2H), 3.27 (s, 3H).

### [Synthesis Example 71 Preparation of N-(4-(5-(methylsulfonyl)benzo[d]oxazol-2-yl)benzyl)nicotinamide hydrochloride (A4510H)

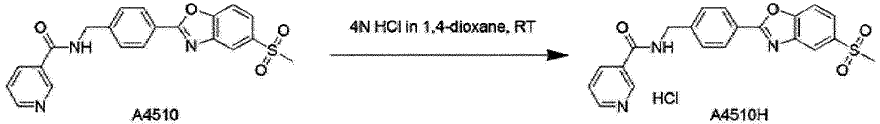

A4510 (1 eq., 4.45 mmol) obtained in Synthesis Example 5 was placed in a 20-ml vial, and 5 ml of a solution of 4M HCl in 1,4-dioxane was added thereto, followed by stirring at room temperature for 4 hours. The reaction product was diluted with ether and then stirred at room temperature for 30 minutes. Thereafter, the product was filtered and dried in a vacuum pump to obtain A4510 hydrochloride (A4510H) as a white solid (yield: 90%).

¹H NMR (400 MHz, DMSO-d₆) δ 9.69 (s, 1H), 9.23 (s, 1H), 8.88 (d, J = 4.1 Hz, 1H), 8.67 - 8.60 (m, 1H), 8.31 (d, J = 1.4 Hz, 1H), 8.19 (d, J = 8.2 Hz, 2H), 8.04 (d, J = 8.6 Hz, 1H), 7.96 (dd, J = 8.6, 1.7 Hz, 1H), 7.87 - 7.79 (m, 1H), 7.60 (d, J = 8.2 Hz, 2H), 4.61 (d, J = 5.8 Hz, 2H), 3.27 (s, 3H).

### [Synthesis Example 81 Preparation of N-(4-(5-methylbenzo[d]oxazol-2-yl)benzyl)nicotinamide (A4275)

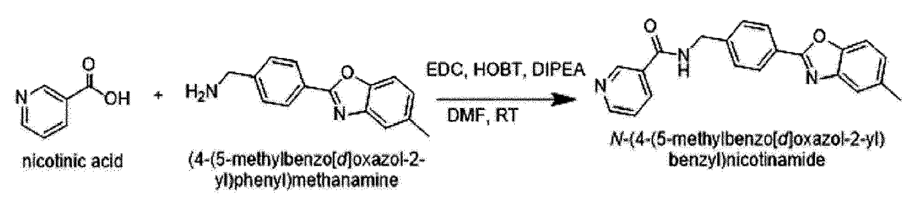

Nicotinic acid (2.44 mmol), (4-(5-methylbenzo[d]oxazol-2-yl)phenyl)methanamine (2.44 mmol), 1-(3-dimethylaminopropyl)-3-ethylcarbodiimide hydrochloride (EDC, 4.87 mmol), 1-hydroxybenzotriazole monohydrate (HOBt, 4.87 mmol) and N,N-diisopropylethylamine (DIPEA, 7.31 mmol) were dissolved in dimethylformamide (DMF, 10 ml) and then refluxed overnight at room temperature. Then, the reaction mixture was transferred to a separate funnel, and then an aqueous NH₄Cl solution was added thereto, followed by extraction three times with ethyl acetate (EA). The extract was dried over Na₂SO₄ and concentrated under reduced pressure. The residue was purified by flash column chromatography (5% methanol in dichloromethane) and then dried in a vacuum pump to obtain N-(4-(5-methylbenzo[d]oxazol-2-yl)benzyl)nicotinamide (A4275).

¹H NMR (400 MHz, DMSO-d₆) δ 9.36 (t, J = 5.5 Hz, 1H), 9.09 (s, 1H), 8.76 - 8.73 (m, 1H), 8.27 (d, J = 7.9 Hz, 1H), 8.17 (d, J = 8.1 Hz, 2H), 7.65 (d, J = 8.3 Hz, 1H), 7.61 - 7.51 (m, 4H), 7.24 (d, J = 8.2 Hz, 1H), 4.62 (d, J = 5.6 Hz, 2H), 2.45 (s, 3H)

### [Synthesis Example 91 Preparation of N-(4-(5-ethylbenzo[d]oxazol-2-yl)benzyl)nicotinamide (A4508)

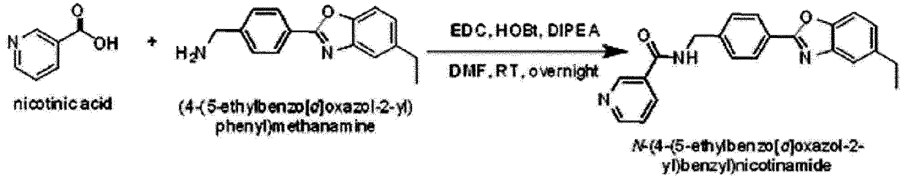

Nicotinic acid (2.44 mmol), (4-(5-ethylbenzo[d]oxazol-2-yl)phenyl)methanamine (2.44 mmol), 1-(3-dimethylaminopropyl)-3-ethylcarbodiimide hydrochloride (EDC, 4.87 mmol), 1-hydroxybenzotriazole monohydrate (HOBt, 4.87 mmol), and N,N-diisopropylethylamine (DIPEA, 7.31 mmol) were dissolved in dimethylformamide (DMF, 10 ml) and then refluxed overnight at room temperature. Then, the reaction mixture was transferred to a separate funnel, and then an aqueous NH₄Cl solution was added thereto, followed by extraction three times with ethyl acetate (EA). The extract was dried over Na₂SO₄ and concentrated under reduced pressure. The residue was purified by flash column chromatography (5% methanol in dichloromethane) and then dried in a vacuum pump to obtain a final compound (hereinafter referred to as "A4508").

### [Synthesis Examples 10 to 301

### <Reaction Scheme 1>

According to Reaction Scheme 1 above, the acid (2.44 mmol) and amine (2.44 mmol) shown in Table 1 below, 1-(3-dimethylaminopropyl)-3-ethylcarbodiimide hydrochloride (EDC, 4.87 mmol), 1-hydroxybenzotriazole monohydrate (HOBt, 4.87 mmol), and N,N-diisopropylethylamine (DIPEA, 7.31 mmol) were dissolved in dimethylformamide (DMF, 10 ml) and then refluxed overnight at room temperature. Then, the reaction mixture was transferred to a separate funnel, and then an aqueous NH₄Cl solution was added thereto, followed by extraction three times with ethyl acetate (EA). The extract was dried over Na₂SO₄ and concentrated under reduced pressure. The residue was purified by flash column chromatography (5% methanol in dichloromethane) and then dried in a vacuum pump, thereby preparing the final compounds shown in Table 2 below.

**[Table 1]**

| Synthesis Example | Acid | Amine |
|---|---|---|
| Synthesis Example 10 | 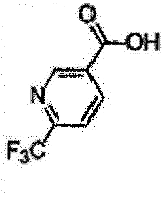 | 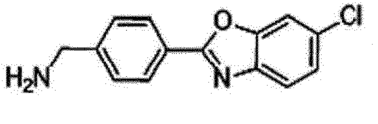 |
| Synthesis Example 11 | 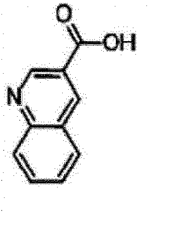 | 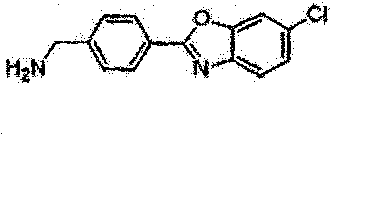 |
| Synthesis Example 12 | 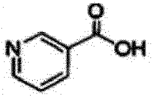 | 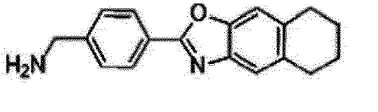 |
| Synthesis Example 13 | 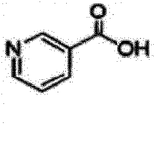 | 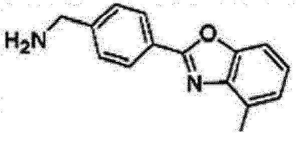 |
| Synthesis Example 14 | 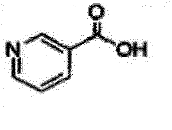 | 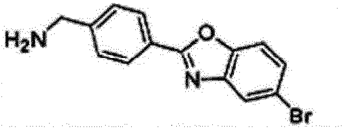 |
| Synthesis Example 15 | 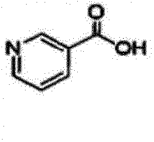 | 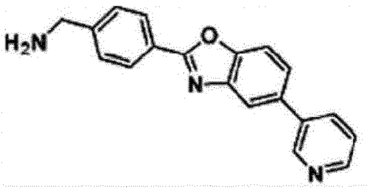 |
| Synthesis Example 16 | 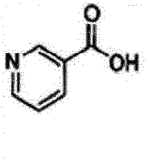 | 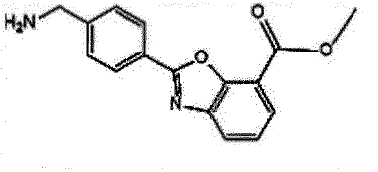 |
| Synthesis Example 17 | 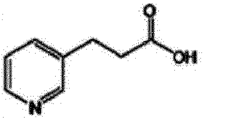 | 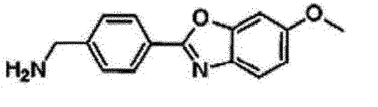 |
| Synthesis Example 18 | 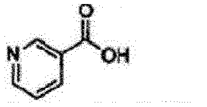 | 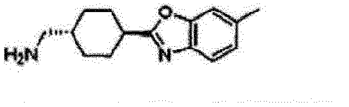 |
| Synthesis Example 19 | 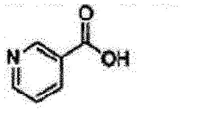 | 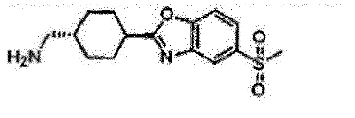 |
| Synthesis Example 20 | 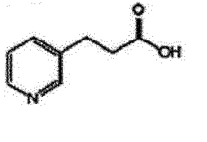 | 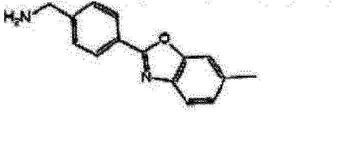 |
| Synthesis Example 21 | 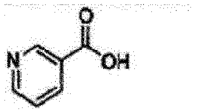 | 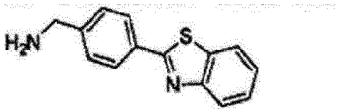 |
| Synthesis Example 22 | 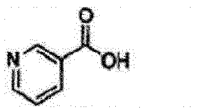 | 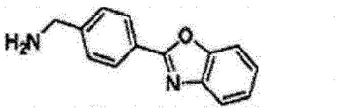 |
| Synthesis Example 23 | 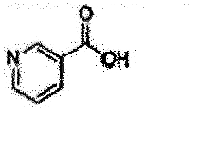 | 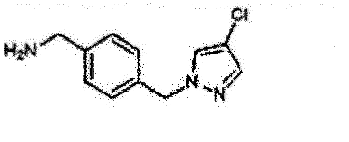 |
| Synthesis Example 24 | 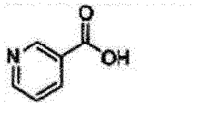 | 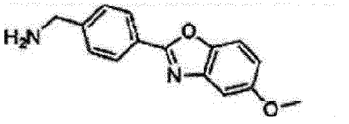 |
| Synthesis Example 25 | 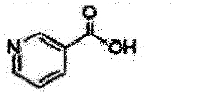 | 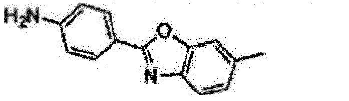 |
| Synthesis Example 26 | 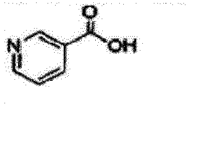 | 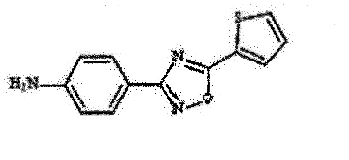 |
| Synthesis Example 27 | 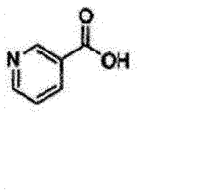 | 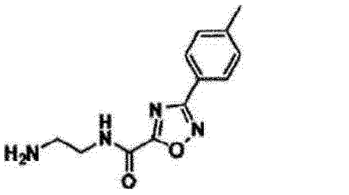 |
| Synthesis Example 28 | 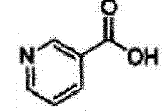 | 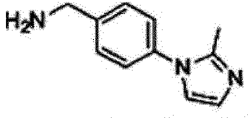 |
| Synthesis Example 29 | 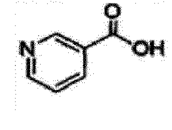 | 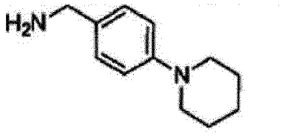 |
| Synthesis Example 30 | 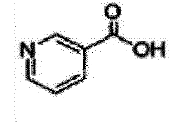 | 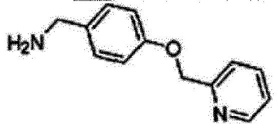 |

**[Table 2]**

| Synthesis Example | Compound Name | Final Compound |
|---|---|---|
| Synthesis Example 10 | A4307 | 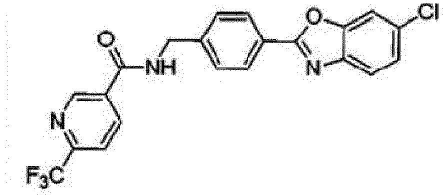 |
| | | *N*-(4-(6-chlorobenzo[*d*]oxazol-2-yl)benzyl)-6-(trifluoromethyl)nicotinamide |
| Synthesis Example 11 | A4308 | *N-*(4-(6-methylbenzo[*d*]oxazol-2-yl) benzyl)benzamide |
| | | 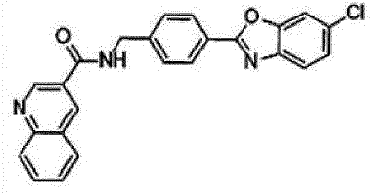 |
| Synthesis Example 12 | A4366 | 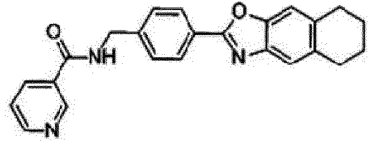 |
| | | *N*-(4-(5,6,7,8-tetrahydronaphtho[2,3-*d*]oxazol-2-yl)benzyl)nicotinamde |
| Synthesis Example 13 | A4367 | 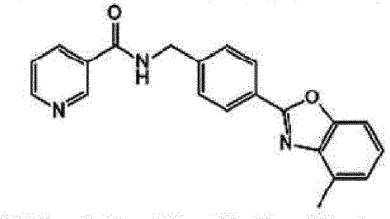 |
| | | *N-(*4-(4-methylbenzo[d]oxazol-2-yl)benzyl)nicotinamide |
| Synthesis Example 14 | A4375 | 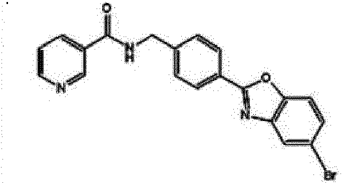 |
| | | *N*-(4-(5-bromobenzo[d]axazol-2-yl)benzyl)nicotinamide |
| Synthesis Example 15 | A4376 | 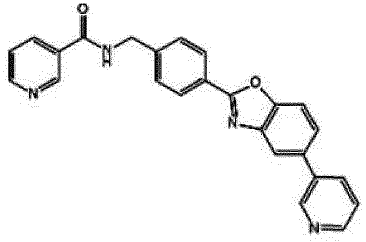 |
| | | *N-*(4-(5-(pyridin-3-yl)benzo[*d*]oxazol-2-yl)benzyl)nicotinamide |
| Synthesis Example 16 | A4511 | 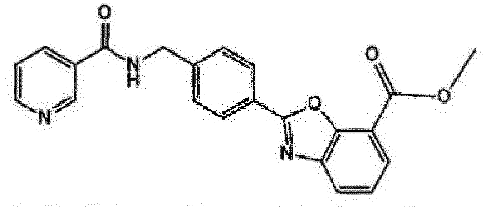 |
| | | methyl 2-(4-(nicotinamidomethyl)phenyl)benzo[*d*]oxazole-7-carboxylate |
| Synthesis Example 17 | A4512 | 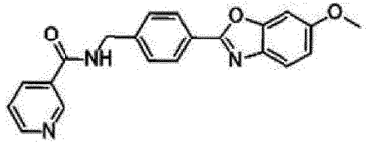 |
| | | *N*-(4-(6-methoxybenzo[d]oxazol-2-yl)benzyl)nicotinamide |
| Synthesis Example 18 | A4560 | 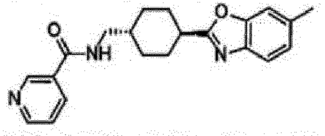 |
| | | *N*-(((1r,4*r*)-4-(6-methylbenzo[*d*]oxazol-2-yl)cyclohexyl)methyl)nicotinamide |
| Synthesis Example 19 | A4562 | 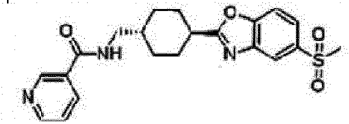 |
| | | *N-(*((1*r*,4*r*)-4-(5-(methylsulfonyl)benzo[d]oxazol-2-yl)cyclohexyl)methyl)nicotinamide |
| Synthesis Example 20 | A4635 | 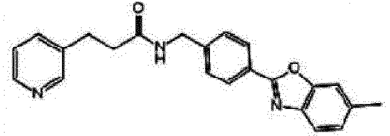 |
| | | N-(4-(6-methylbenzo[*d*]oxazol-2-yl)benzyl)-3-(pyridin-3-yl)propanamide |
| Synthesis Example 21 | B1468 | 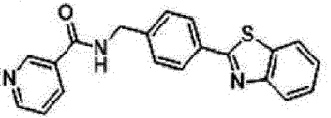 |
| | | *N-*(4-(benzo[*d*]thiazol-2-yl)benzyl)nicotinamide |
| Synthesis Example 22 | B1470 | 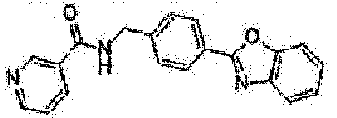 |
| | | *N-(*4-(benzo[d]oxazol-2-yl)benzyl)nicotinamide |
| Synthesis Example 23 | B1473 | 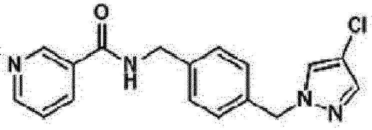 |
| | | N-(4-((4-chloro-1H-pyrazol4-yl)methyl)benzyl)niocotinamide |
| Synthesis Example 24 | B1474 | 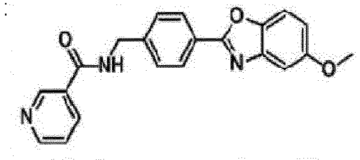 |
| | | *N*-(4-(5-methoxybenzo[*d*]oxazol-2-yl)benzyl)nicotinamide |
| Synthesis Example 25 | B1481 | 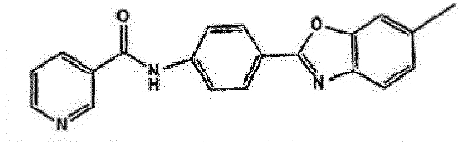 |
| | | *N-*(4-(6-methylbenzo[d]oxazol-2-yl)phenyl)nicotinamide |
| Synthesis Example 26 | B1482 | 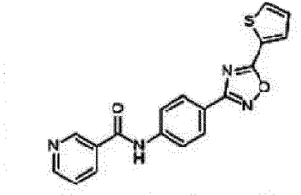 |
| | | N-(4-(5-(thiophen-2-yl)-1,,2,4-oxadiazol-3-yl)phenyl)nicotinamide |
| Synthesis Example 27 | B1857 | 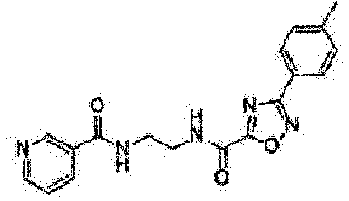 |
| | | *N*-(2-(nicotinamido)ethyl)-3-(*p-*tolyl) -1,2,4-oxadiazole-5-carboxamide |
| Synthesis Example 28 | B1967 | 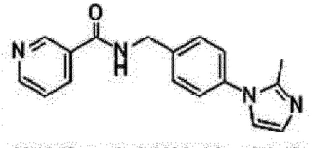 |
| | | *N*-(4-(2-methyl-1*H*-imidazol-1-yl) benzyl)nlcotinamide |
| Synthesis Example 29 | B1978 | 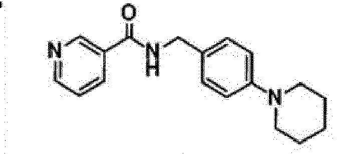 |
| | | *N*-(4-(piperidin-1-yl)benzyl)nicotinamide |
| Synthesis Example 30 | B1979 | 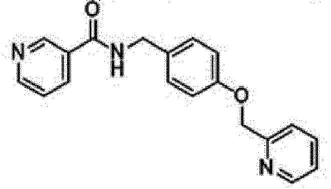 |
| | | *N*-(4-(pyridin-2-ylmethoxy)benzyl)nicotinamide |

### [Experimental Example 11 Evaluation of the ability to inhibit SARM1 enzymethat triggers axonal degeneration (1)

As described above, it has been reported that SARM1 promotes axonal degeneration through MKK4-JNK as a downstream signaling in a traumatic axonal injury model (Yang et al., Cell 2015, PMID 25594179), and that SARM1 promotes JNK phosphorylation, and as a result, promotes immune responses around injured neurons (Wang et al., Cell Reports 2018, PMID 29669278). In addition, it has been reported that the JNK pathway is required for axonal degeneration, and that this axonal degeneration is delayed upon JNK inhibition (Miller et al., Nature Neuroscience volume 12, pages 387-389(2009); PNAS December 26, 2012, 109 (52) 21199-21200).

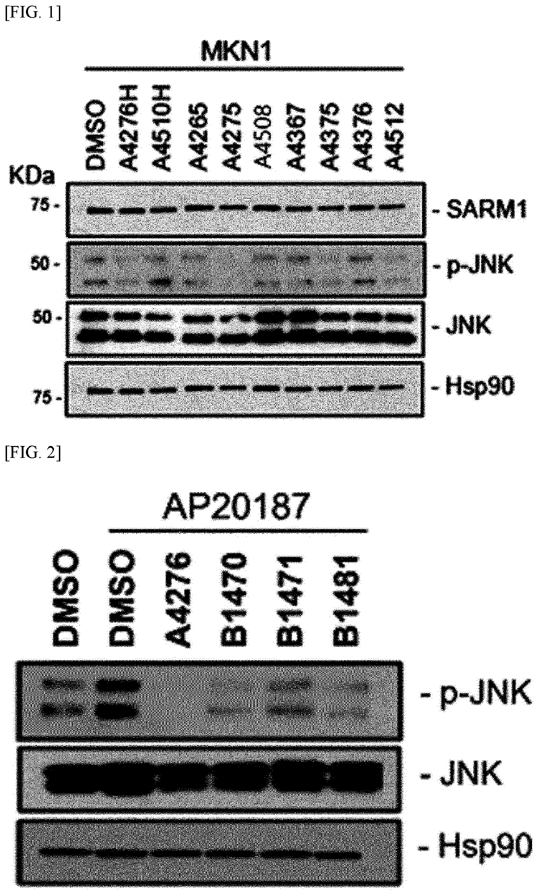

Accordingly, in order to evaluate the axonal degeneration inhibitory effects of the compounds according to the present invention, the abilities of these compounds to inhibit SARM1 enzyme were analyzed. Specifically, whether phosphorylation of JNK protein, a representative sub-regulatory marker of SARM1, decreased, was evaluated by Western blot assay. A method for preparing the protein used in the Western blot assay is as follows. The gastric cancer cell line MKN1 was treated with PBS, harvested, washed with PBS, and then lysed using RIPA buffer as a cell lysis buffer containing each of A4276H, A4510H, A4265, A4275, A4367, A4375, A4376 and A4512 prepared in the Synthesis Examples. The entire cell lysate was fractionated using a centrifuge, and then only the solution containing the protein was extracted. The extracted protein was quantified by the Bradford method. The protein of the same concentration obtained through quantification was separated by SDS-polyacrylamide gel electrophoresis, and then transferred to a PVDF membrane. The membrane to which the protein has been transferred was blocked with a TBS-T (Tris-buffered saline/0.1% Tween-20) solution containing 5% non-fat milk at room temperature for 1 hour in order to reduce nonspecific binding, and incubated with a primary antibody (diluted in 5% BSA solution) at 4°C for 12 hours or more, and then incubated with a secondary antibody (1:5000 dilution) at room temperature for 1 hour. For visualization, an enhanced chemiluminescence system was used. Western blot assay was performed using JNK protein, a representative marker whose phosphorylation is regulated by SARM1, and the housekeeping protein Hsp90 was used for quantitative comparison of expression. The results are shown in FIG. 1.

As shown in FIG. 1, it could be confirmed that all of the compounds according to the present invention inhibited JNK phosphorylation. On the other hand, although not shown in the figure, it could be seen that N-(4-(6-chlorobenzo[d]oxazol-2-yl)benzyl)nicotinamide (A4266) also had the effect of inhibiting JNK phosphorylation. These compounds are characterized by commonly having a nicotinamide group and a benzoxazole group at both ends.

### [Experimental Example 21 Evaluation of the ability to inhibit SARM1 enzyme that triggers axonal degeneration (2)

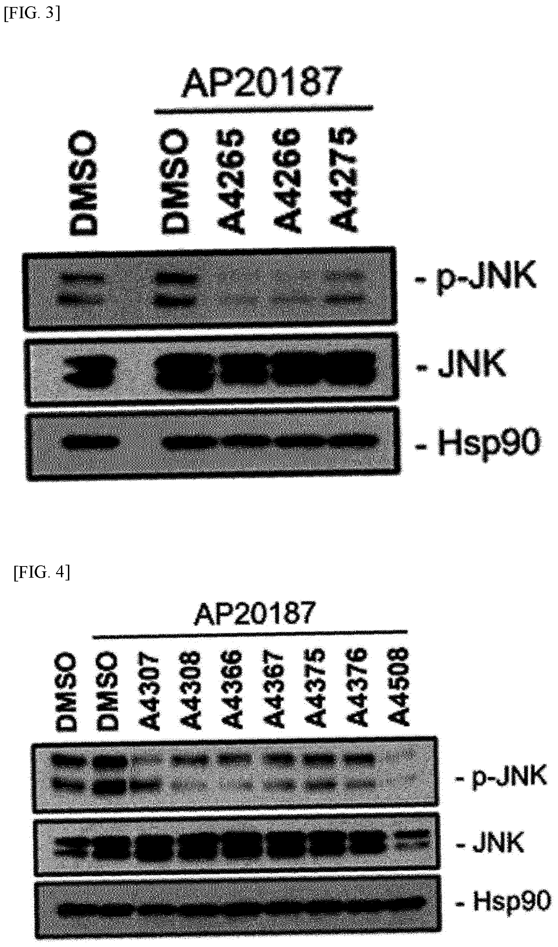

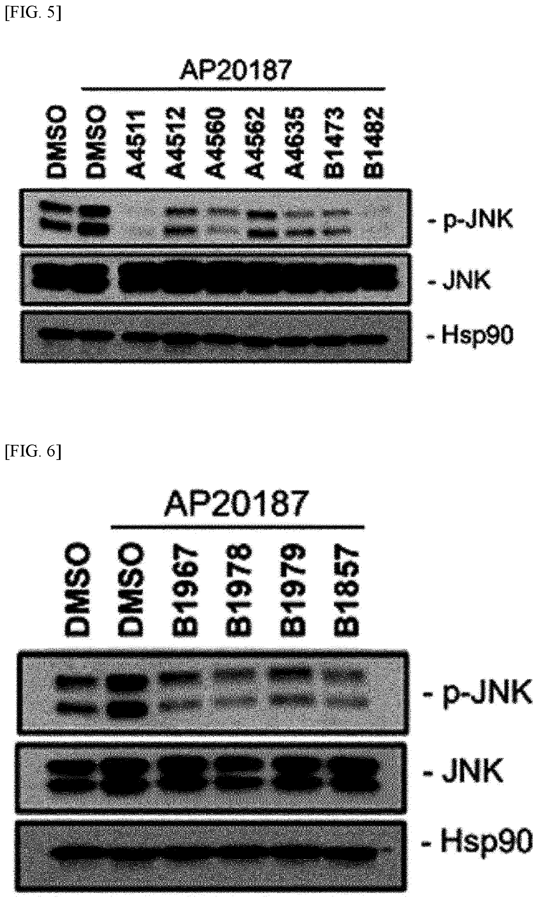

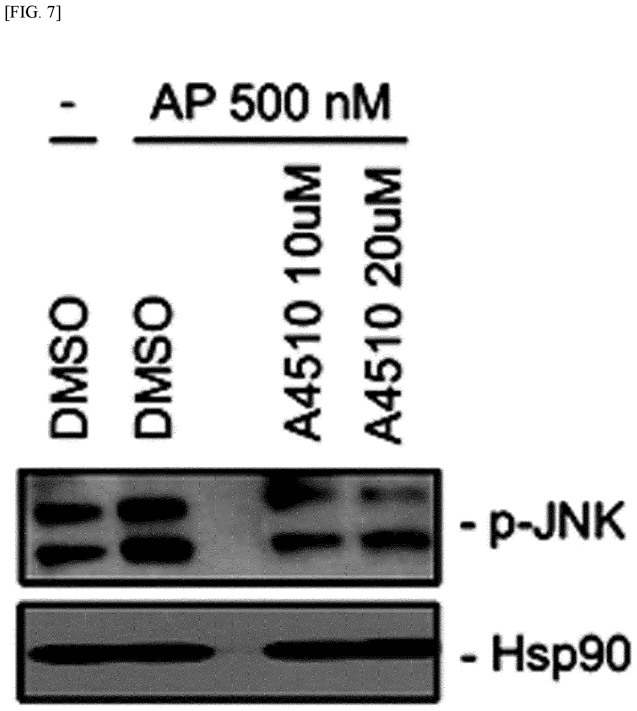

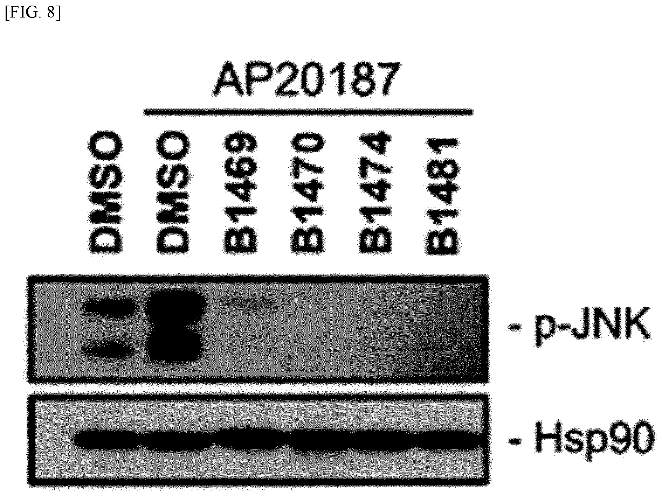

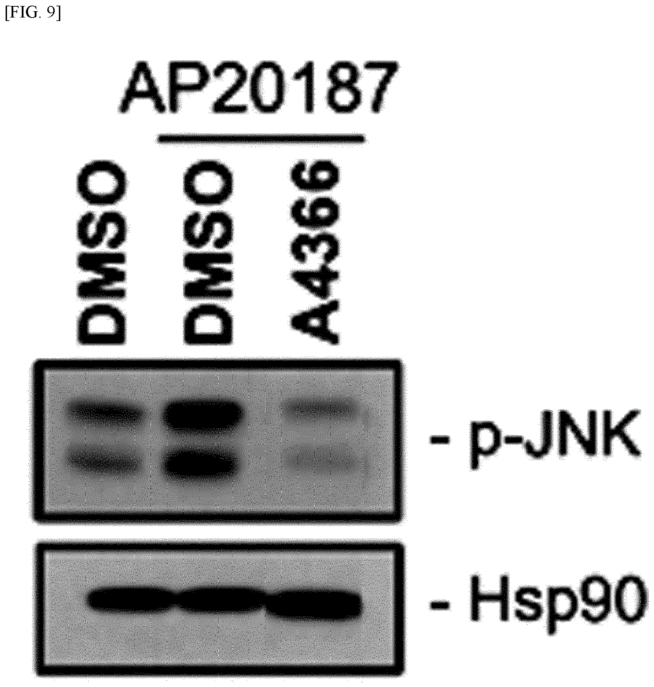

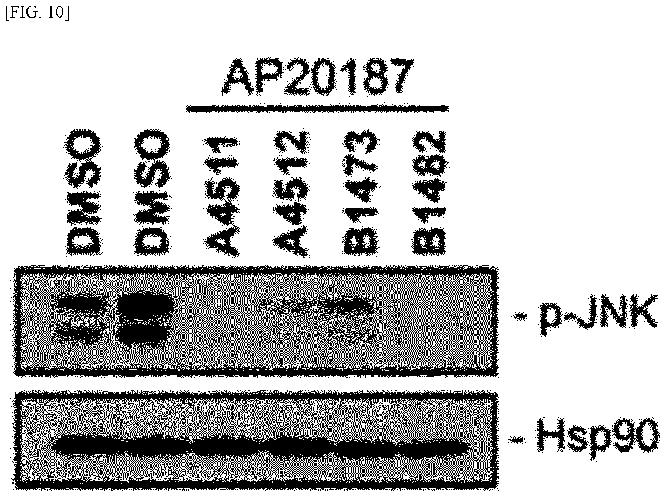

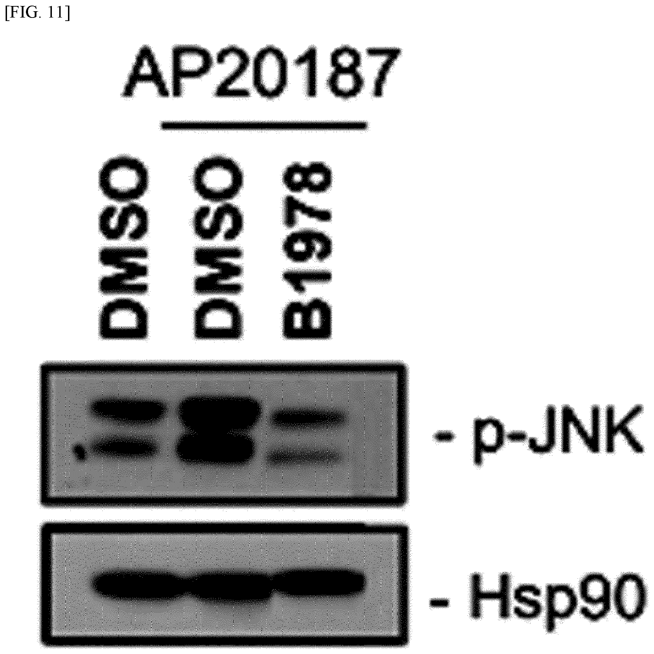

24 hours before the experiment, the F11 cell line (a fusion of the mouse neuroblastoma cell line N18TG-2 with embryonic rat dorsal-root ganglion (DRG) neurons) was seeded into 6-well plates at a density of 3 x 10⁵ cell/well. Using Lipofectamine 2000 reagent, each well was transfected with 1 µg of a pCMV-FKBPF36V-TIR vector. The reason for attaching the FKBP domain (FKBPF36V) to the TIR is that the dimerization of the FKBP domain can be artificially induced upon administration of the AP20187 substance, which in turn induces dimerization of and activates the SARM1 TIR domain attached thereto. After 24 hours, each well was pretreated with each of the compounds according to the present invention at a concentration of 10 µM for 1 hour, and then treated with 500 nM AP20187 for activation of the TIR domain. After 24 hours, each well was washed with cold PBS and then lysed using RIPA buffer. Next, the protein was quantified by the Bradford assay and then sampled using SDS buffer. 10 µg of the sample was loaded on 12% SDS-PAGE gel. The protein-loaded gel was transferred to a PVDF membrane, incubated in a blocking solution (5% skim milk) for 1 hour, and then subjected to an antigen-antibody reaction with a primary antibody (diluted 1:1,000) at 4°C for 12 hours or more. After unbound antibody was removed by washing three times with 1x TBST for 10 minutes, the gel was subjected to an antigen-antibody reaction with a secondary antibody (diluted 1:3,000) at room temperature for 1 hour. In addition, after unbound antibody was removed by washing three times with 1x TBST for 10 minutes, a color reaction was developed using the ECL solution to visualize the band. The results are shown in FIGS. 2 to 12.

As shown in FIGS. 2 to 12, it could be seen that all of the compounds according to the present invention had the effect of inhibiting JNK phosphorylation, and the inhibited phosphorylation of the JNK protein was not restored by AP20187, suggesting that the effect of the compounds on the inhibition of JNK phosphorylation was the effect obtained by the inhibition of SARM1 or the inhibition of a downstream pathway thereof. For reference, AP20187 is known as a substance that activates the FKBP domain by promoting the dimerization thereof (Yang et al., Cell 2015, PMID 25594179; Wang et al., Cell Reports 2018, PMID 29669278).

### [Experimental Example 31 Preparation of tadpole optic nerve axotomy model and drug efficacy experiment (1)

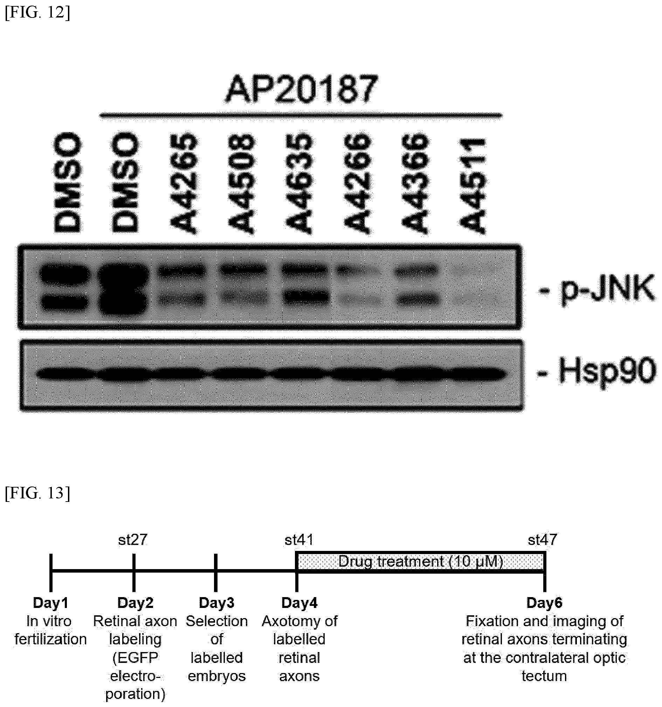

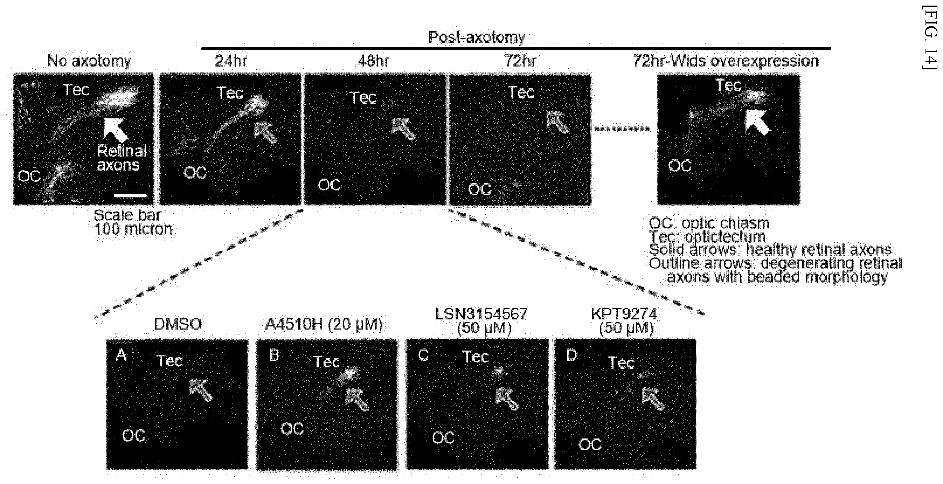

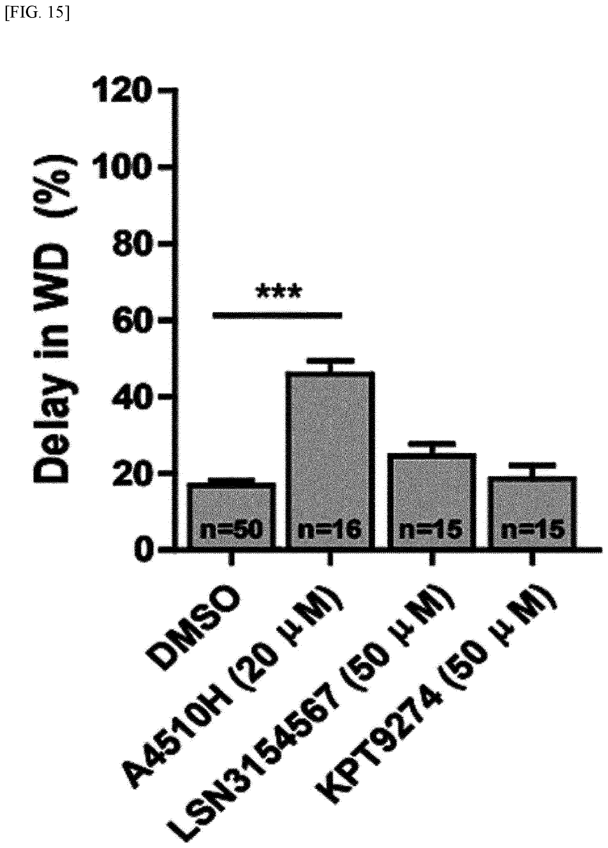

In order to verify the axon protective effect of the compound according to the present invention in the tadpole optic nerve axotomy model, an experiment was conducted according to the design shown in FIG. 13. Specifically, an *in vitro* fertilized Xenopus tropicalis (African frog) embryo (Nasco, Fort Atkinson, WI 53538, USA) is cultured to stage 27, and then a plasmid DNA expressing Enhanced Green Fluorescence Protein (EGFP) is delivered into the retinal progenitor cells of one eye by an electroporation technique. After culturing to stage 41, the axons of the retinal ganglion cells expressing the green fluorescent protein reach the optic tectum of the contralateral brain hemisphere, and this axon bundle is referred to as the optic nerve. When the optic nerve is axotomized at stage 41, Wallerian degeneration occurs in which axons distal from the cell body degenerate by fragmention over about 72 hours. While the optic nerve was axotomized, the embryo was treated with various drugs, including N-(4-(5-(methylsulfonyl)benzo[d]oxazol-2-yl)benzyl)nicotinamide (A4510), and LSN 3154567 and KPT9274 as controls. 48 hours after treatment, the embryo was fixed in a solution of 4% paraformaldehyde in phosphate buffered saline. After extraction of the brain of the fixed embryo, the green fluorescent protein signal of the optic nerve in the brain hemisphere was imaged using a confocal microscope (model LSM700, Carl Zeiss, Oberkochen, Germany), and the results are shown in FIG. 14. Wallerian degeneration of the Xenopus optic nerve occurr in a stereotypical pattern at 24 hours, 48 hours and 72 hours after axotomy, and the degree of delay in Wallerian degeneration was quantified as percentage by comparing the degree of Wallerian degeneration in the drug-treated group with this pattern. The results of the quantification are shown in FIG. 15.

As shown in FIGS. 14 and 15, it could be seen that LSN 3154567 and KPT9274 used as controls did not exhibit a significant axon protective effect, but the compound according to the present invention exhibited an excellent axon protective effect.

### [Experimental Example 41 Preparation of tadpole optic nerve axotomy model and drug efficacy experiment (2)

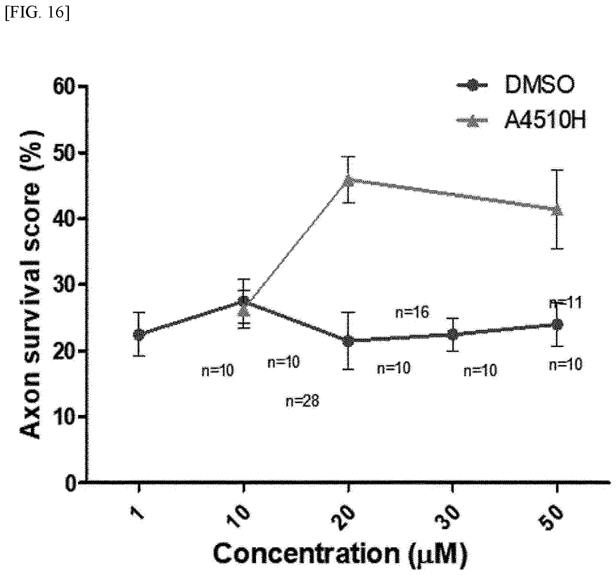

In order to verify the axon protective effect of the compound according to the present invention in the tadpole optic nerve axotomy model, an experiment was conducted in the same manner as in Experimental Example 3. Specifically, GFP-expressing DNA was delivered into one retina of a Xenopus tropicalis tadpole at stage 27 (before retinal development) by electroporation. At stage 43 (the time when the connection between the retina and the visual center of the brain is finished), axons of the epectroporated retina were axotomized, and the tissue was fixed at 24-hour intervals, followed by extraction of the brain. Next, the ventral midline of the brain was excised and unfolded, and then the GFP signal in the contra-lateral brain hemisphere was imaged with a confocal microscope (LSM700, Carl Zeiss, 20x, z-step 3 µm). Thereby, it was possible to observe retinal ganglion cell axons located along the optic tract extending from the optic chiasm to the optic tectum. A non-axotomized uninterrupted GFP signal could be observed, which was defined as a healthy axon. Axonal degeneration could be quantified because the continuous GFP signal disappeared along the optic tract over time after axotomy. In most cases, compared to normal tissue, about 50% healthy axons, about 10% healthy axons and 0% healthy axons appeared after 24 hours, 48 hours and 72 hours, respectively. The degree of inhibition of axonal degeneration by the drug was evaluated 48 hours after axotomy. As the drug, N-(4-(5-(methylsulfonyl)benzo[d] oxazol-2-yl) benzyl)nicotinamide hydrochloride (A4510H) was used, and as a control, DMSO was used. Treatment with the drug or the control was performed simultaneously with axotomy, and the non-axotomized group was included in each experiment. The percentage of healthy axons in the drug group relative to the control group taken as 100% was calculated and used as the axon survival score (0 to 100%). The results are shown in FIG. 16.

As shown in FIG. 16, it could be seen that N-(4-(5-(methylsulfonyl)benzo[d]oxazol-2-yl)benzyl)nicotinamide hydrochloride (A4510H) according to the present invention exhibited an excellent axon protective effect.

### [Experimental Example 51 Evaluation of inhibitory effect against axonal degeneration caused by anticancer drugs

The dorsal root ganglias (DRGs) of mouse embryos (1 to 5 days old) were separated into cell units, and then each cell was fluidically divided into the cell body and the axon by culture in a microfluidic device. At this time, only the axon region was treated with each of the compounds (drugs) of Synthesis Examples 1 to 30 according to the present invention, and movement of the treated drug from the axon region to the cell body region was prevented by increasing the amount of the cell body region medium compared to the amount of the axon region medium through application of pressure due to the difference in height between the media. Treatment with the drug was performed 24 hours before treatment with the anticancer drug vincristine or paclitaxel. The experiment was conducted using a total of three experimental groups: a control group (Untreated), a vincristine/paclitaxel-treated group, and a drug-treated group (40 nM vincristine/100 nM paclitaxel + 2 µM test drug). 24 hours after drug treatment, fixing with 4% paraformaldehyde (PFA) was performed. Immunocytochemistry (ICC) was performed with acetylation-alpha-tubulin, and axonal degeneration was quantitatively analyzed by imaging the degree of axonal degeneration with a fluorescence microscope.

As a result, although not shown in the drawings, it could be confirmed that, when treatment with the compound according to the present invention was performed, axonal degeneration was inhibited even when subsequent treatment with an anticancer agent such as vincristine or paclitaxel was performed.

Thereby, it could be seen that that the compound of the present invention could exhibit the effect of inhibiting axonal degeneration, and could also effectively prevent or treat chemotherapy-induced peripheral neuropathy (CIPN) by protecting axons from injury caused by anticancer drugs.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The present invention is directed to a composition capable of preventing or inhibiting axonal degeneration and effectively preventing, ameliorating or treating various neurological diseases caused by axonal degeneration.

## Claims

1. A pharmaceutical composition for preventing or inhibiting axonal degeneration containing, as an active ingredient, a compound selected from among a compound represented by the following Formula (1), and a pharmaceutically acceptable salt, optical isomer, hydrate and solvate thereof: 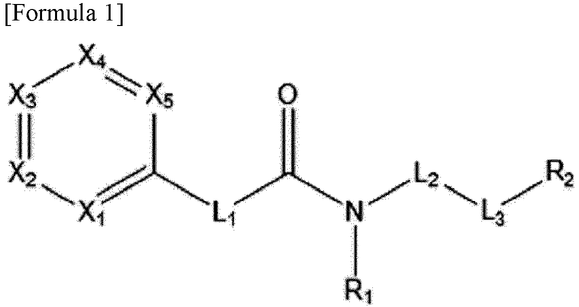 wherein
X₁ to X₅ are each independently N or C(R₃), provided that at least one of X₁ to X₅ is N;
L₁ is a direct bond or a C₁-C₆ alkylene group;
L₂ and L₃ are each independently selected from the group consisting of a direct bond, a C₁-C₆ alkylene group, a C₃-C₇ cycloalkylene group, a heterocycloalkylene group having 5 to 7 nuclear atoms, a C₆-C₁₄ arylene group, a heteroarylene group having 5 to 14 nuclear atoms, a divalent C₅-C₁₄ non-aromatic fused polycyclic group, and a divalent non-aromatic fused heteropolycyclic group having 5 to 14 nuclear atoms;
R₁ is selected from the group consisting of hydrogen, a C₁-C₆ alkyl group, a C₃-C₇ cycloalkyl group, and a heterocycloalkyl group having 5 to 7 nuclear atoms;
R₂ is a group represented by the following Formula 2;
R₃ is selected from the group consisting of hydrogen, a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₃ is present in a plural number, the plurality of R₃ are the same as or different from each other, or the plurality of R₃ present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkylene group in L₁ to L₃, the cycloalkylene group, heterocycloalkylene group, arylene group, heteroarylene group, divalent non-aromatic fused polycyclic group and divalent non-aromatic fused heteropolycyclic group in L₂ and L₃, and the alkyl group, cycloalkyl group and heterocycloalkyl group in R₁ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other;
the alkyl group, alkoxy group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₃, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₃ adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other; 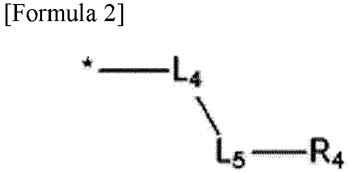 wherein
^{∗} denotes a bonding position;
L₄ and L₅ are each independently selected from the group consisting of a direct bond, a C₁-C₆ alkylene group, a C₆-C₁₄ arylene group, a heteroarylene group having 5 to 14 nuclear atoms, ^{∗}-(CH₂)a-O-(CH₂)b-^{∗}, a carbonyl group (^{∗}-C(=O)-^{∗}), ^{∗}-N(R₅)-^{∗}, and an amide group (^{∗}-C(=O)-N(H)-^{∗} or ^{∗}-N(H)-C(=O)-^{∗});
a and b are each independently an integer ranging from 0 to 6;
R₄ is selected from the group consisting of a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, and ^{∗}-N(R₆)(R₇);
R₅ is selected from the group consisting of hydrogen, a C₁-C₆ alkyl group, a C₃-C₇ cycloalkyl group, and a heterocycloalkyl group having 5 to 7 nuclear atoms;
R₆ and R₇ are each independently selected from the group consisting of hydrogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, or they bond to each other to form a heterocycloalkyl ring having 5 to 7 nuclear atoms, a heteroaryl ring having 5 to 14 nuclear atoms, or a non-aromatic fused heteropolycyclic ring having 5 to 14 nuclear atoms;
the alkyl group, cycloalkyl group and heterocycloalkyl group in R₅ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
the alkylene group, arylene group and heteroarylene group in L₄ and L₅, the cycloalkyl group, heterocycloalkyl group, aryl group, heteroaryl group, non-aromatic fused polycyclic group and non-aromatic fused heteropolycyclic group in R₄, R₆ and R₇, the alkyl group in R₆ and R₇, and the heterocycloalkyl ring, heteroaryl ring and non-aromatic fused heteropolycyclic ring, which are formed by bonding between R₆ and R₇, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other, or the plurality of substituents present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms.

2. The pharmaceutical composition of claim 1, wherein the compound represented by Formula 1 is a compound represented by the following Formula 3: 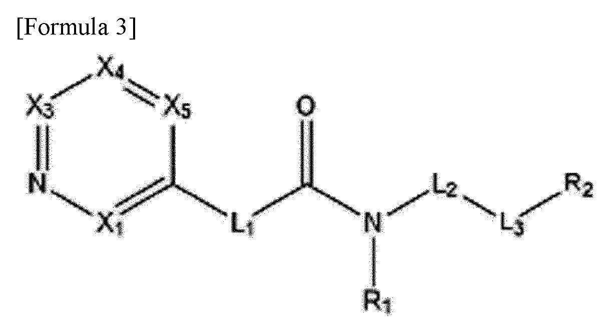 wherein
X₁, X₃ to X₅, L₁ to L₃, R₁ and R₂ are as defined in claim 1.

3. The pharmaceutical composition of claim 1, wherein the compound represented by Formula 1 is a compound represented by the following Formula 6: 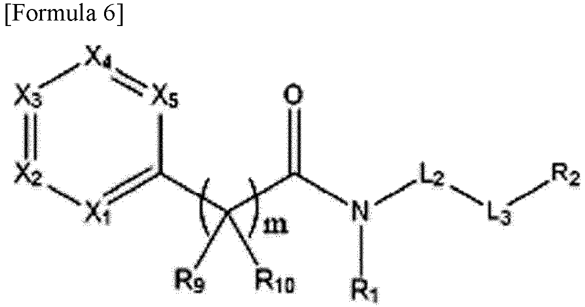 wherein
m is an integer ranging from 0 to 6;
R₉ and R₁₀ are each independently selected from the group consisting of hydrogen, a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when m is an integer ranging from 2 to 6, the plurality of R₉ are the same as or different from each other and the plurality of Rio are the same as or different from each other;
the alkyl group, cycloalkyl group, heterocycloalkyl group, aryl group, heteroaryl group, non-aromatic fused polycyclic group and non-aromatic fused heteropolycyclic group in R₉ and R₁₀ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
X₁ to X₅, L₂, L₃, R₁ and R₂ are as defined in claim 1.

4. The pharmaceutical composition of claim 1, wherein L₂ is a direct bond or a C₁-C₆ alkylene group unsubstituted or substituted with a C₁-C₆ alkyl group; L₃ is a direct bond or is selected from the group consisting of a C₃-C₇ cycloalkylene group, a heterocycloalkylene group having 5 to 7 nuclear atoms, a C₆-C₁₄ arylene group, and a heteroarylene group having 5 to 14 nuclear atoms, and the cycloalkylene group, heterocycloalkylene group, arylene group and heteroarylene group in L₃ are each independently substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms.

5. The pharmaceutical composition of claim 1, wherein the compound represented by Formula 1 is a compound represented by the following Formula 7: 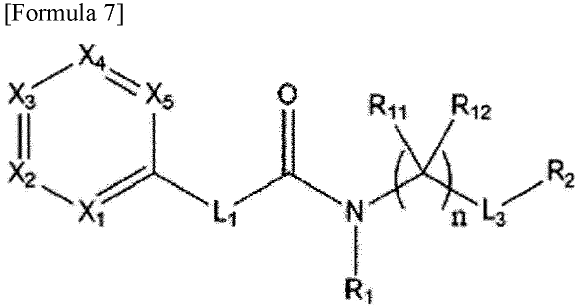 wherein
n is an integer ranging from 0 to 6;
R₁₁ and R₁₂ are each independently selected from the group consisting of hydrogen, a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when n is an integer ranging from 2 to 6, the plurality of R₁₁ are the same as or different from each other and the plurality of R₁₂ are the same as or different from each other;
the alkyl group, cycloalkyl group, heterocycloalkyl group, aryl group, heteroaryl group, non-aromatic fused polycyclic group and non-aromatic fused heteropolycyclic group in R₁₁ and R₁₂ are each independently unsubstituted or substituted with at least one substituent from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
X₁ to X₅, L₁, L₃, R₁ and R₂ are as defined in claim 1.

6. The pharmaceutical composition of claim 1, wherein the compound represented by Formula 1 is a compound represented by the following Formula 8 or 9: 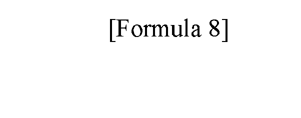 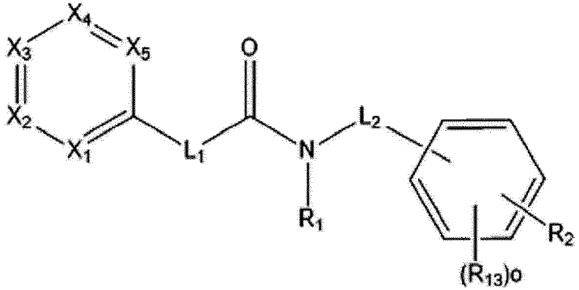 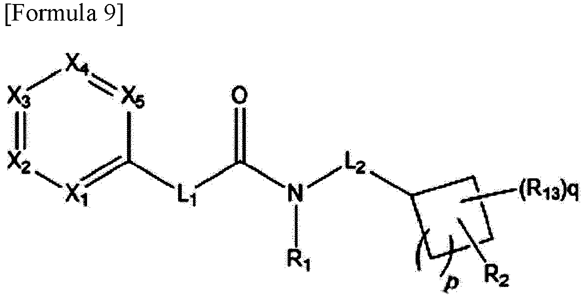 wherein
o and p are each independently an integer ranging from 0 to 4;
q is an integer ranging from 0 to 5;
R₁₃ is selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when R₁₃ is present in a plural number, the plurality of R₁₃ are the same as or different from each other;
the alkyl group, cycloalkyl group, heterocycloalkyl group, aryl group, heteroaryl group, non-aromatic fused polycyclic group and non-aromatic fused heteropolycyclic group in R₁₃ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
X₁ to X₅, L₁, L₂, R₁ and R₂ are as defined in claim 1.

7. The pharmaceutical composition of claim 1, wherein R₄ is a group represented by the following Formula 12: 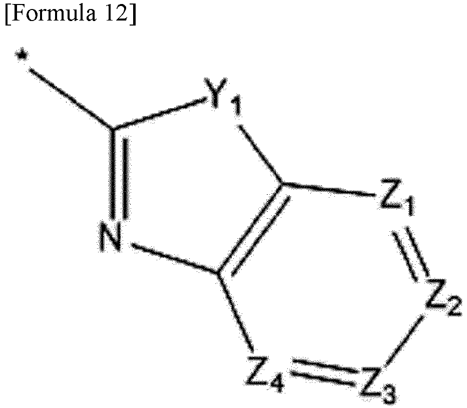 wherein
^{∗} denotes a bonding position;
Y₁ is O or S;
Z₁ to Z₄ are each independently N or C(R₁₄);
R₁₄ is selected from the group consisting of hydrogen, a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₁₄ is present in a plural number, the plurality of R₁₄ are the same as or different from each other, or the plurality of R₁₄ present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkyl group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₁₄, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₁₄ present adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other.

8. The pharmaceutical composition of claim 7, wherein R₄ is represented by any one of the following Formulas a1 to a7: 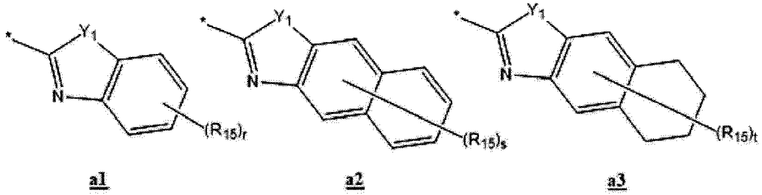 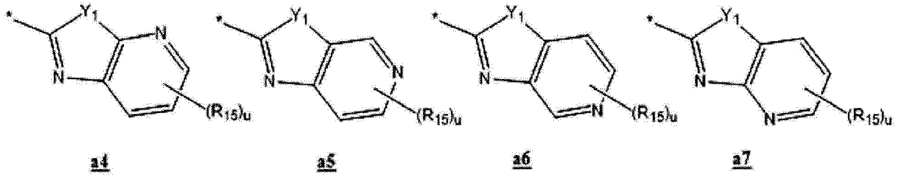 wherein
r is an integer ranging from 0 to 4;
s is an integer ranging from 0 to 6;
t is an integer ranging from 0 to 10;
u is an integer ranging from 0 to 3;
R₁₅ is selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₁₅ is present in a plural number, the plurality of R₁₅ are the same as or different from each other, or the plurality of R₁₅ present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkyl group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₁₅, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₁₅ present adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁ to C₆ alkoxycarbonyl group, and a C₁-C₆ alkylsulfonyl group, and when they are substituted with a plurality of substituents, these substituents are the same as or different from each other; and
Y₁ is as defined in claim 7.

9. The pharmaceutical composition of claim 1, wherein the compound represented by Formula 1 is a compound selected from the group consisting of the following compounds:
N-(4-(6-methylbenzo[d]oxazol-2-yl)benzyl)nicotinamide;
N-(4-(6-chlorobenzo[d]oxazol-2-yl)benzyl)nicotinamide;
N-(4-(naphtho[2,3-d]oxazol-2-yl)benzyl)nicotinamide;
N-(4-(oxazolo[4,5-c]pyridin-2-yl)benzyl)nicotinamide;
N-(4-(5-(methylsulfonyl)benzo[d]oxazol-2-yl)benzyl)nicotinamide;
N-(4-(5-methylbenzo[d]oxazol-2-yl)benzyl)nicotinamide;
N-(4-(5-ethylbenzo[d]oxazol-2-yl)benzyl)nicotinamide;
N-(4-(6-chlorobenzo[d]oxazol-2-yl)benzyl)-6-(trifluoromethyl)nicotinamide;
N-(4-(6-methylbenzo[d]oxazol-2-yl)benzyl)benzamide;
N-(4-(5,6,7,8-tetrahydronaphtho[2,3-d]oxazol-2-yl)benzyl)nicotinamide;
N-(4-(4-methylbenzo[d]oxazol-2-yl)benzyl)nicotinamide;
N-(4-(5-bromobenzo[d]oxazol-2-yl)benzyl)nicotinamide;
N-(4-(5-(pyridin-3-yl)benzo[d]oxazol-2-yl)benzyl)nicotinamide;
methyl 2-(4-(nicotinamidomethyl)phenyl)benzo[d]oxazole-7-carboxylate;
N-(4-(6-methoxybenzo[d]oxazol-2-yl)benzyl)nicotinamide;
N-(((1r,4r)-4-(6-methylbenzo[d]oxazol-2-yl)cyclohexyl)methyl)nicotinamide;
N-(((1r,4r)-4-(5-(methylsulfonyl)benzo[d]oxazol-2-yl)cyclohexyl)methyl)nicotinamide;
N-(4-(6-methylbenzo[d]oxazol-2-yl)benzyl)-3-(pyridin-3-yl)propanamide;
N-(4-(benzo[d]thiazol-2-yl)benzyl)nicotinamide;
N-(4-(benzo[d]oxazol-2-yl)benzyl)nicotinamide;
N-(4-((4-chloro-1H-pyrazol-1-yl)methyl)benzyl)nicotinamide;
N-(4-(5-methoxybenzo[d]oxazol-2-yl)benzyl)nicotinamide;
N-(4-(6-methylbenzo[d]oxazol-2-yl)phenyl)nicotinamide;
N-(4-(5-(thiophen-2-yl)-1,2,4-oxadiazol-3-yl)phenyl)nicotinamide;
N-(2-(nicotinamido)ethyl)-3-(p-tolyl)-1,2,4-oxadiazole-5-carboxamide;
N-(4-(2-methyl-1H-imidazol-1-yl)benzyl)nicotinamide;
N-(4-(piperidin-1-yl)benzyl)nicotinamide; and
N-(4-(pyridin-2-ylmethoxy)benzyl)nicotinamide.

10. A pharmaceutical composition for preventing or inhibiting neurological diseases caused by axonal degeneration containing, as an active ingredient, a compound selected from among a compound represented by the following Formula (1), and a pharmaceutically acceptable salt, optical isomer, hydrate and solvate thereof: 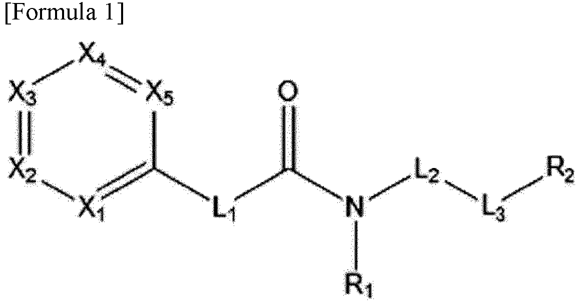 wherein
X₁ to X₅ are each independently N or C(R₃), provided that at least one of X₁ to X₅ is N;
L₁ is a direct bond or a C₁-C₆ alkylene group;
L₂ and L₃ are each independently selected from the group consisting of a direct bond, a C₁-C₆ alkylene group, a C₃-C₇ cycloalkylene group, a heterocycloalkylene group having 5 to 7 nuclear atoms, a C₆-C₁₄ arylene group, a heteroarylene group having 5 to 14 nuclear atoms, a divalent C₅-C₁₄ non-aromatic fused polycyclic group, and a divalent non-aromatic fused heteropolycyclic group having 5 to 14 nuclear atoms;
R₁ is selected from the group consisting of hydrogen, a C₁-C₆ alkyl group, a C₃-C₇ cycloalkyl group, and a heterocycloalkyl group having 5 to 7 nuclear atoms;
R₂ is a group represented by the following Formula 2;
R₃ is selected from the group consisting of hydrogen, a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₃ is present in a plural number, the plurality of R₃ are the same as or different from each other, or the plurality of R₃ present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkylene group in L₁ to L₃, the cycloalkylene group, heterocycloalkylene group, arylene group, heteroarylene group, divalent non-aromatic fused polycyclic group and divalent non-aromatic fused heteropolycyclic group in L₂ and L₃, and the alkyl group, cycloalkyl group and heterocycloalkyl group in R₁ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other;
the alkyl group, alkoxy group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₃, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₃ adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other; 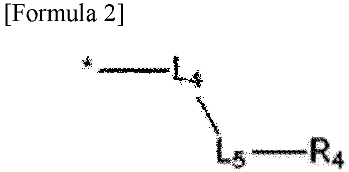 wherein
^{∗} denotes a bonding position;
L₄ and L₅ are each independently selected from the group consisting of a direct bond, a C₁-C₆ alkylene group, a C₆-C₁₄ arylene group, a heteroarylene group having 5 to 14 nuclear atoms, ^{∗}-(CH₂)a-O-(CH₂)b-^{∗}, a carbonyl group (^{∗}-C(=O)-^{∗}), ^{∗}-N(R₅)-^{∗}, and an amide group (^{∗}-C(=O)-N(H)-^{∗} or ^{∗}-N(H)-C(=O)-^{∗});
a and b are each independently an integer ranging from 0 to 6;
R₄ is selected from the group consisting of a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, and ^{∗}-N(R₆)(R₇);
R₅ is selected from the group consisting of hydrogen, a C₁-C₆ alkyl group, a C₃-C₇ cycloalkyl group, and a heterocycloalkyl group having 5 to 7 nuclear atoms;
R₆ and R₇ are each independently selected from the group consisting of hydrogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, or they bond to each other to form a heterocycloalkyl ring having 5 to 7 nuclear atoms, a heteroaryl ring having 5 to 14 nuclear atoms, or a non-aromatic fused heteropolycyclic ring having 5 to 14 nuclear atoms;
the alkyl group, cycloalkyl group and heterocycloalkyl group in R₅ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
the alkylene group, arylene group and heteroarylene group in L₄ and L₅, the cycloalkyl group, heterocycloalkyl group, aryl group, heteroaryl group, non-aromatic fused polycyclic group and non-aromatic fused heteropolycyclic group in R₄, R₆ and R₇, the alkyl group in R₆ and R₇, and the heterocycloalkyl ring, heteroaryl ring and non-aromatic fused heteropolycyclic ring, which are formed by bonding between R₆ and R₇, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other, or the plurality of substituents present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms.

11. The pharmaceutical composition of claim 10, wherein the compound represented by Formula 1 is a compound represented by the following Formula 3: 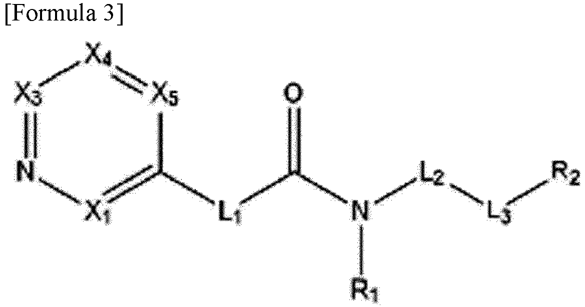 wherein
X₁, X₃ to X₅, L₁ to L₃, R₁ and R₂ are as defined in claim 10.

12. The pharmaceutical composition of claim 10, wherein the compound represented by Formula 1 is a compound represented by the following Formula 6: 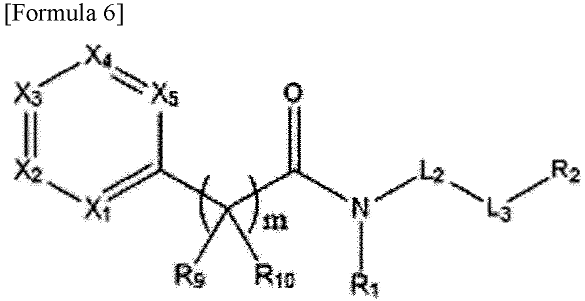 wherein
m is an integer ranging from 0 to 6;
R₉ and R₁₀ are each independently selected from the group consisting of hydrogen, a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when m is an integer ranging from 2 to 6, the plurality of R₉ are the same as or different from each other and the plurality of Rio are the same as or different from each other;
the alkyl group, cycloalkyl group, heterocycloalkyl group, aryl group, heteroaryl group, non-aromatic fused polycyclic group and non-aromatic fused heteropolycyclic group in R₉ and R₁₀ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
X₁ to X₅, L₂, L₃, R₁ and R₂ are as defined in claim 10.

13. The pharmaceutical composition of claim 10, wherein L₂ is a direct bond or a C₁-C₆ alkylene group unsubstituted or substituted with a C₁-C₆ alkyl group; L₃ is a direct bond or is selected from the group consisting of a C₃-C₇ cycloalkylene group, a heterocycloalkylene group having 5 to 7 nuclear atoms, a C₆-C₁₄ arylene group, and a heteroarylene group having 5 to 14 nuclear atoms, and the cycloalkylene group, heterocycloalkylene group, arylene group and heteroarylene group in L₃ are each independently substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms.

14. The pharmaceutical composition of claim 10, wherein the compound represented by Formula 1 is a compound represented by the following Formula 7: 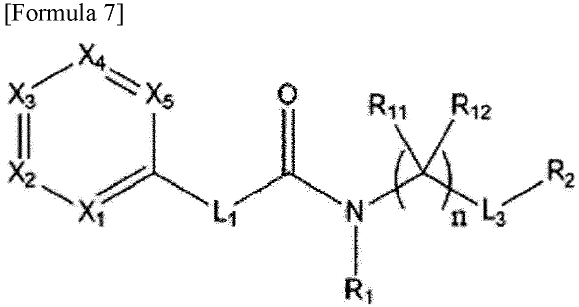 wherein
n is an integer ranging from 0 to 6;
R₁₁ and R₁₂ are each independently selected from the group consisting of hydrogen, a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when n is an integer ranging from 2 to 6, the plurality of R₁₁ are the same as or different from each other and the plurality of R₁₂ are the same as or different from each other;
the alkyl group, cycloalkyl group, heterocycloalkyl group, aryl group, heteroaryl group, non-aromatic fused polycyclic group and non-aromatic fused heteropolycyclic group in R₁₁ and R₁₂ are each independently unsubstituted or substituted with at least one substituent from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
X₁ to X₅, L₁, L₃, R₁ and R₂ are as defined in claim 10.

15. The pharmaceutical composition of claim 10, wherein the compound represented by Formula 1 is a compound represented by the following Formula 8 or 9: 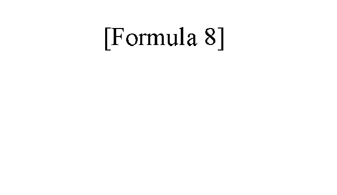 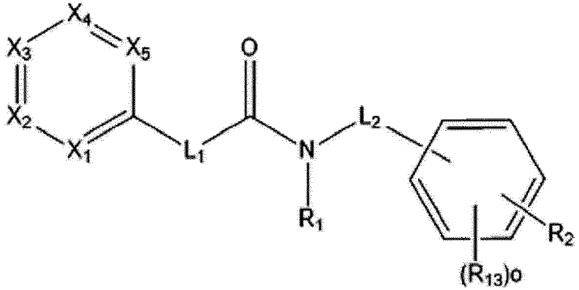 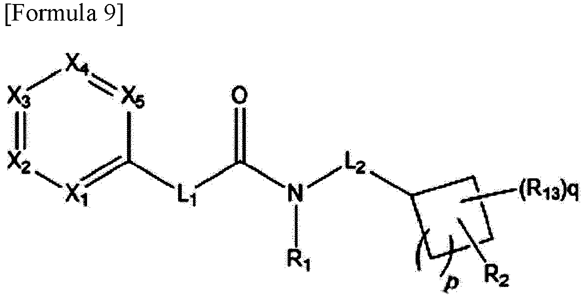 wherein
o and p are each independently an integer ranging from 0 to 4;
q is an integer ranging from 0 to 5;
R₁₃ is selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when R₁₃ is present in a plural number, the plurality of R₁₃ are the same as or different from each other;
the alkyl group, cycloalkyl group, heterocycloalkyl group, aryl group, heteroaryl group, non-aromatic fused polycyclic group and non-aromatic fused heteropolycyclic group in R₁₃ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
X₁ to X₅, L₁, L₂, R₁ and R₂ are as defined in claim 10.

16. The pharmaceutical composition of claim 10, wherein R₄ is a group represented by the following Formula 12: 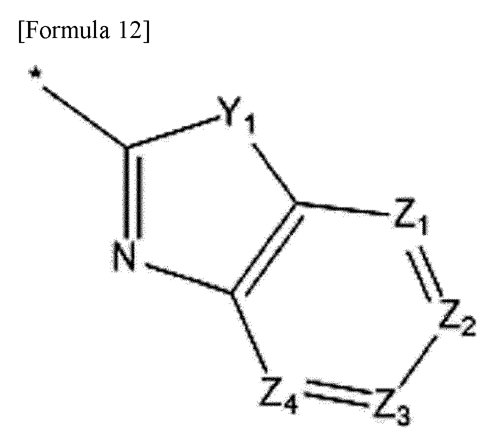 wherein
^{∗} denotes a bonding position;
Y₁ is O or S;
Z₁ to Z₄ are each independently N or C(R₁₄);
R₁₄ is selected from the group consisting of hydrogen, a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₁₄ is present in a plural number, the plurality of R₁₄ are he same as or different from each other, or the plurality of R₁₄ present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkyl group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₁₄, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₁₄ present adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other.

17. The pharmaceutical composition of claim 16, wherein R₄ is represented by any one of the following Formulas a1 to a7: 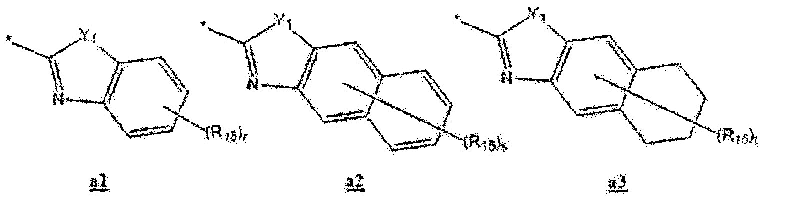 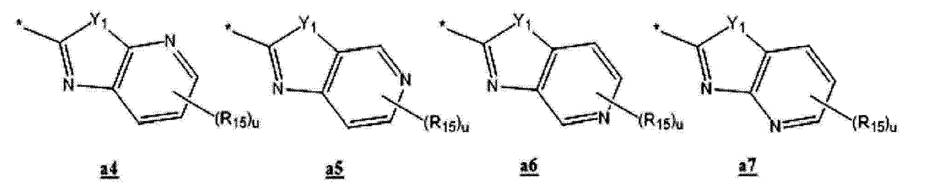 wherein
r is an integer ranging from 0 to 4;
s is an integer ranging from 0 to 6;
t is an integer ranging from 0 to 10;
u is an integer ranging from 0 to 3;
R₁₅ is selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic , a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₁₅ is present in a plural number, the plurality of R₁₅ are the same as or different from each other, or the plurality of R₁₅ present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkyl group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₁₅, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₁₅ present adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁ to C₆ alkoxycarbonyl group, and a C₁-C₆ alkylsulfonyl group, and when they are substituted with a plurality of substituents, these substituents are the same as or different from each other; and
Y₁ is as defined in claim 16.

18. The pharmaceutical composition of claim 10, wherein the compound represented by Formula 1 is a compound selected from the group consisting of the following compounds:
N-(4-(6-methylbenzo[d]oxazol-2-yl)benzyl)nicotinamide;
N-(4-(6-chlorobenzo[d]oxazol-2-yl)benzyl)nicotinamide;
N-(4-(naphtho[2,3-d]oxazol-2-yl)benzyl)nicotinamide;
N-(4-(oxazolo[4,5-c]pyridin-2-yl)benzyl)nicotinamide;
N-(4-(5-(methylsulfonyl)benzo[d]oxazol-2-yl)benzyl)nicotinamide;
N-(4-(5-methylbenzo[d]oxazol-2-yl)benzyl)nicotinamide;
N-(4-(5-ethylbenzo[d]oxazol-2-yl)benzyl)nicotinamide;
N-(4-(6-chlorobenzo[d]oxazol-2-yl)benzyl)-6-(trifluoromethyl)nicotinamide;
N-(4-(6-methylbenzo[d]oxazol-2-yl)benzyl)benzamide;
N-(4-(5,6,7,8-tetrahydronaphtho[2,3-d]oxazol-2-yl)benzyl)nicotinamide;
N-(4-(4-methylbenzo[d]oxazol-2-yl)benzyl)nicotinamide;
N-(4-(5-bromobenzo[d]oxazol-2-yl)benzyl)nicotinamide;
N-(4-(5-(pyridin-3-yl)benzo[d]oxazol-2-yl)benzyl)nicotinamide; methyl 2-(4-(nicotinamidomethyl)phenyl)benzo[d]oxazole-7-carboxylate;
N-(4-(6-methoxybenzo[d]oxazol-2-yl)benzyl)nicotinamide;
N-(((1r,4r)-4-(6-methylbenzo[d]oxazol-2-yl)cyclohexyl)methyl)nicotinamide;
N-(((1r,4r)-4-(5-(methylsulfonyl)benzo[d]oxazol-2-yl)cyclohexyl)methyl)nicotinamide;
N-(4-(6-methylbenzo[d]oxazol-2-yl)benzyl)-3-(pyridin-3-yl)propanamide;
N-(4-(benzo[d]thiazol-2-yl)benzyl)nicotinamide;
N-(4-(benzo[d]oxazol-2-yl)benzyl)nicotinamide;
N-(4-((4-chloro-1H-pyrazol-1-yl)methyl)benzyl)nicotinamide;
N-(4-(5-methoxybenzo[d]oxazol-2-yl)benzyl)nicotinamide;
N-(4-(6-methylbenzo[d]oxazol-2-yl)phenyl)nicotinamide;
N-(4-(5-(thiophen-2-yl)-1,2,4-oxadiazol-3-yl)phenyl)nicotinamide;
N-(2-(nicotinamido)ethyl)-3-(p-tolyl)-1,2,4-oxadiazole-5-carboxamide;
N-(4-(2-methyl-1H-imidazol-1-yl)benzyl)nicotinamide;
N-(4-(piperidin-1-yl)benzyl)nicotinamide; and
N-(4-(pyridin-2-ylmethoxy)benzyl)nicotinamide.

19. A method for preventing or inhibiting axonal degeneration comprising a step of administering, to a subject in need of administration, a pharmaceutically effective amount of a compound selected from among a compound represented by the following Formula (1), and a pharmaceutically acceptable salt, optical isomer, hydrate and solvate thereof: 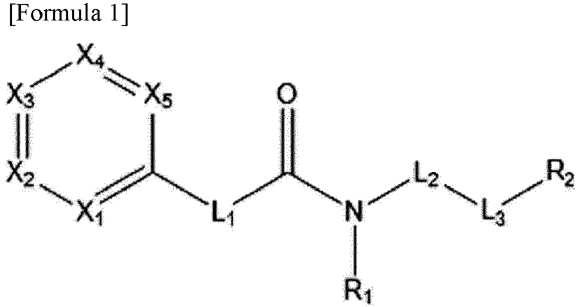 wherein
X₁ to X₅ are each independently N or C(R₃), provided that at least one of X₁ to X₅ is N;
L₁ is a direct bond or a C₁-C₆ alkylene group;
L₂ and L₃ are each independently selected from the group consisting of a direct bond, a C₁-C₆ alkylene group, a C₃-C₇ cycloalkylene group, a heterocycloalkylene group having 5 to 7 nuclear atoms, a C₆-C₁₄ arylene group, a heteroarylene group having 5 to 14 nuclear atoms, a divalent C₅-C₁₄ non-aromatic fused polycyclic group, and a divalent non-aromatic fused heteropolycyclic group having 5 to 14 nuclear atoms;
R₁ is selected from the group consisting of hydrogen, a C₁-C₆ alkyl group, a C₃-C₇ cycloalkyl group, and a heterocycloalkyl group having 5 to 7 nuclear atoms;
R₂ is a group represented by the following Formula 2;
R₃ is selected from the group consisting of hydrogen, a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₃ is present in a plural number, the plurality of R₃ are the same as or different from each other, or the plurality of R₃ present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkylene group in L₁ to L₃, the cycloalkylene group, heterocycloalkylene group, arylene group, heteroarylene group, divalent non-aromatic fused polycyclic group and divalent non-aromatic fused heteropolycyclic group in L₂ and L₃, and the alkyl group, cycloalkyl group and heterocycloalkyl group in R₁ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other;
the alkyl group, alkoxy group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₃, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₃ adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other; 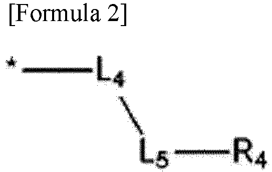 wherein
^{∗} denotes a bonding position;
L₄ and L₅ are each independently selected from the group consisting of a direct bond, a C₁-C₆ alkylene group, a C₆-C₁₄ arylene group, a heteroarylene group having 5 to 14 nuclear atoms, ^{∗}-(CH₂)a-O-(CH₂)b-^{∗}, a carbonyl group (^{∗}-C(=O)-^{∗}), ^{∗}-N(R₅)-^{∗}, and an amide group (^{∗}-C(=O)-N(H)-^{∗} or ^{∗}-N(H)-C(=O)-^{∗});
a and b are each independently an integer ranging from 0 to 6;
R₄ is selected from the group consisting of a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, and ^{∗}-N(R₆)(R₇);
R₅ is selected from the group consisting of hydrogen, a C₁-C₆ alkyl group, a C₃-C₇ cycloalkyl group, and a heterocycloalkyl group having 5 to 7 nuclear atoms;
R₆ and R₇ are each independently selected from the group consisting of hydrogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, or they bond to each other to form a heterocycloalkyl ring having 5 to 7 nuclear atoms, a heteroaryl ring having 5 to 14 nuclear atoms, or a non-aromatic fused heteropolycyclic ring having 5 to 14 nuclear atoms;
the alkyl group, cycloalkyl group and heterocycloalkyl group in R₅ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
the alkylene group, arylene group and heteroarylene group in L₄ and L₅, the cycloalkyl group, heterocycloalkyl group, aryl group, heteroaryl group, non-aromatic fused polycyclic group and non-aromatic fused heteropolycyclic group in R₄, R₆ and R₇, the alkyl group in R₆ and R₇, and the heterocycloalkyl ring, heteroaryl ring and non-aromatic fused heteropolycyclic ring, which are formed by bonding between R₆ and R₇, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other, or the plurality of substituents present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms.

20. A method for preventing or treating neurological diseases caused by axonal degeneration comprising a step of administering, to a subject in need of administration, a pharmaceutically effective amount of a compound selected from among a compound represented by the following Formula (1), and a pharmaceutically acceptable salt, optical isomer, hydrate and solvate thereof: 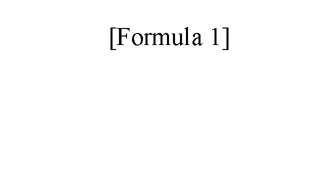 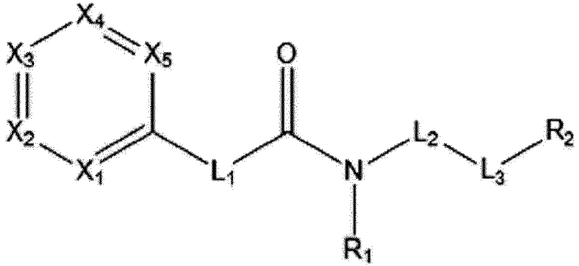 wherein
X₁ to X₅ are each independently N or C(R₃), provided that at least one of X₁ to X₅ is N;
L₁ is a direct bond or a C₁-C₆ alkylene group;
L₂ and L₃ are each independently selected from the group consisting of a direct bond, a C₁-C₆ alkylene group, a C₃-C₇ cycloalkylene group, a heterocycloalkylene group having 5 to 7 nuclear atoms, a C₆-C₁₄ arylene group, a heteroarylene group having 5 to 14 nuclear atoms, a divalent C₅-C₁₄ non-aromatic fused polycyclic group, and a divalent non-aromatic fused heteropolycyclic group having 5 to 14 nuclear atoms;
R₁ is selected from the group consisting of hydrogen, a C₁-C₆ alkyl group, a C₃-C₇ cycloalkyl group, and a heterocycloalkyl group having 5 to 7 nuclear atoms;
R₂ is a group represented by the following Formula 2;
R₃ is selected from the group consisting of hydrogen, a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when R₃ is present in a plural number, the plurality of R₃ are the same as or different from each other, or the plurality of R₃ present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms;
the alkylene group in L₁ to L₃, the cycloalkylene group, heterocycloalkylene group, arylene group, heteroarylene group, divalent non-aromatic fused polycyclic group and divalent non-aromatic fused heteropolycyclic group in L₂ and L₃, and the alkyl group, cycloalkyl group and heterocycloalkyl group in R₁ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other;
the alkyl group, alkoxy group, cycloalkyl group, heterocycloalkyl group, aryl group, heteroaryl group, non-aromatic fused polycyclic group, non-aromatic fused heteropolycyclic group, alkoxycarbonyl group and alkylsulfonyl group in R₃, and the cycloalkyl ring, heterocycloalkyl ring, aryl ring, heteroaryl ring, non-aromatic fused polycyclic ring and non-aromatic fused heteropolycyclic ring, which are formed by bonding between the plurality of R₃ adjacent to each other, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other; 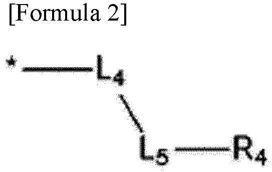 wherein
^{∗} denotes a bonding position;
L₄ and L₅ are each independently selected from the group consisting of a direct bond, a C₁-C₆ alkylene group, a C₆-C₁₄ arylene group, a heteroarylene group having 5 to 14 nuclear atoms, ^{∗}-(CH₂)a-O-(CH₂)b-^{∗}, a carbonyl group (^{∗}-C(=O)-^{∗}), ^{∗}-N(R₅)-^{∗}, and an amide group (^{*-}C(=O)-N(H)-^{*} or ^{∗}-N(H)-C(=O)-^{∗});
a and b are each independently an integer ranging from 0 to 6;
R₄ is selected from the group consisting of a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, and ^{∗}-N(R₆)(R₇);
R₅ is selected from the group consisting of hydrogen, a C₁-C₆ alkyl group, a C₃-C₇ cycloalkyl group, and a heterocycloalkyl group having 5 to 7 nuclear atoms;
R₆ and R₇ are each independently selected from the group consisting of hydrogen, a C₁-C₆ alkyl group, a C₃-C₇ cycloalkyl group, a heterocycloalkyl group having 5 to 7 nuclear atoms, a C₆ to C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, or they bond to each other to form a heterocycloalkyl ring having 5 to 7 nuclear atoms, a heteroaryl ring having 5 to 14 nuclear atoms, or a non-aromatic fused heteropolycyclic ring having 5 to 14 nuclear atoms;
the alkyl group, cycloalkyl group and heterocycloalkyl group in R₅ are each independently unsubstituted or substituted with at least one substituent selected from the group consisting of a halogen, a C₁-C₆ alkyl group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, and a non-aromatic fused heteropolycyclic group having 5 to 14 nuclear atoms, and when they are substituted with a plurality of substituents, these substituents are the same as or different from each other; and
the alkylene group, arylene group and heteroarylene group in L₄ and L₅, the cycloalkyl group, heterocycloalkyl group, aryl group, heteroaryl group, non-aromatic fused polycyclic group and non-aromatic fused heteropolycyclic group in R₄, R₆ and R₇, the alkyl group in R₆ and R₇, and the heterocycloalkyl ring, heteroaryl ring and non-aromatic fused heteropolycyclic ring, which are formed by bonding between R₆ and R₇, are each independently unsubstituted or substituted with at least one substituent selected from the group consisting of a halogen, a C₁-C₆ alkyl group, a C₁-C₆ alkoxy group, a C₃-C₇ cycloalkoxy group, a C₃-C₇ cycloalkyl group, a heterocycloalkyl group having 5 to 7 nuclear atoms, a C₆-C₁₄ aryl group, a heteroaryl group having 5 to 14 nuclear atoms, a C₅-C₁₄ non-aromatic fused polycyclic group, a non-aromatic fused heteropolycyclic group having 5 to 14 nuclear atoms, a carboxy group (^{∗}-C(=O)-OH), a C₁-C₆ alkoxycarbonyl group, and a C₁-C₆ alkylsulfonyl group, and when they are substituted with a plurality of substituents, these substituents are the same as or different from each other, or the plurality of substituents present adjacent to each other bond to each other to form a C₃-C₇ cycloalkyl ring, a heterocycloalkyl ring having 5 to 7 nuclear atoms, a C₆-C₁₄ aryl ring, a heteroaryl ring having 5 to 14 nuclear atoms, a C₅-C₁₄ non-aromatic fused polycyclic ring, or a non-aromatic fused heteropolycyclic ring having 5 to 14 nuclear atoms.
